# EUROPEAN PATENT APPLICATION

(11) **EP 4 722 375 A1**
(43) Date of publication of application: **08.04.2026**
(21) Application number: 24461625.6
(22) Date of filing: 07.10.2024
(51) Int. Cl.: C12P 19/34, C12N 9/12, A61K 31/7115, A61K 31/712, A61K 31/7125

(54) **EFFICIENT IN VITRO TRANSCRIPTION OF ARTIFICIAL MRNA WITH HIGHLY MODIFIED 5' ENDS**

(71) Applicant: ExploRNA Therapeutics Sp. z o. o., 02-089 Warszawa (PL)
(72) Inventor: KOWALSKA, Joanna, 02-089 WARSZAWA (PL); SMIETANSKI, Miroslaw, 02-089 WARSZAWA (PL); BEDNARCZYK, Marcelina, 02-089 WARSZAWA (PL); MLYNARSKA-CIESLAK, Agnieszka, 02-089 WARSZAWA (PL); BARANOWSKI, Marek, 02-089 WARSZAWA (PL); PISAREK, Sabina, 02-089 WARSZAWA (PL); WOLOSEWICZ, Karol, 02-089 WARSZAWA (PL); MAJEWSKI, Bartosz, 02-089 WARSZAWA (PL); GOLAB, Jakub, 02-089 WARSZAWA (PL); NOWIS, Dominika, 02-089 WARSZAWA (PL); JEMIELITY, Jacek, 02-089 WARSZAWA (PL)
(74) Representative: Eisenführ Speiser

(57) **Abstract**

The present invention relates to a molecular complex suitable for in vitro transcription with a T7 RNA polymerase comprising modified tetranucleotide cap analogs, wherein the cap analog may include modified nucleobases. The invention further relates to a complex comprising a specific T7 promoter with a specific trinucleotide initiating sequence and a hybridizing tetranucleotide cap analog. The invention also relates to a use of said molecular complex for in vitro transcription of mRNA with high capping efficiency. Further, the invention relates to a method of producing at least one m7G-capped mRNA molecule. The invention also relates to a pharmaceutical composition for use in a method of treating and/or preventing a disease, comprising said at least one m7G-capped mRNA molecule.

## Description

### Technical Field

The present invention relates to a molecular complex suitable for in vitro transcription with a T7 RNA polymerase comprising modified tetranucleotide cap analogs, wherein the cap analog may include modified nucleobases. The invention further relates to a complex comprising a specific T7 promoter with a specific trinucleotide initiating sequence and a hybridizing tetranucleotide cap analog. The invention also relates to a use of said molecular complex for in vitro transcription of mRNA with high capping efficiency. Further, the invention relates to a method of producing at least one m⁷G-capped mRNA molecule. The invention also relates to a pharmaceutical composition for use in a method of treating and/or preventing a disease, comprising said at least one m⁷G-capped mRNA molecule.

### Background of the invention

### Introduction

The advancement of in vitro transcription (IVT) of mRNA has revolutionized the field of molecular biology and therapeutic development, presenting a pivotal technology in modern biotechnology and medicine. The efficient production of mRNA in vitro is paramount for a myriad of applications, including vaccine development, gene therapy, and synthetic biology. As the demand for rapid and scalable production of mRNA grows, the need for highly efficient IVT systems becomes increasingly critical.

In vitro transcription of mRNA refers to the process by which RNA polymerases synthesize mRNA from a DNA template in a controlled laboratory setting. This technology overcomes the limitations of traditional cellular transcription with the promise for precise control over the mRNA sequence, yield, and purity. IVT systems are particularly crucial in the context of mRNA vaccines, which have demonstrated immense potential in combating infectious diseases, as evidenced by the recent success of mRNA-based COVID-19 vaccines (Polack et al., Safety and Efficacy of the BNT162b2 mRNA Covid-19 Vaccine, N Engl J Med (2020), 383, 27, 2603-2615).

The efficiency of in vitro mRNA transcription directly impacts the scalability and cost-effectiveness of mRNA production. Efficient IVT processes result in higher yields of mRNA, reducing the amount of raw materials and time required. This is especially important for large-scale manufacturing needed to meet the global demand for mRNA therapeutics and vaccines. Additionally, efficient IVT systems minimize the presence of impurities and undesirable by-products, which enhances the safety and efficacy of the final mRNA product.

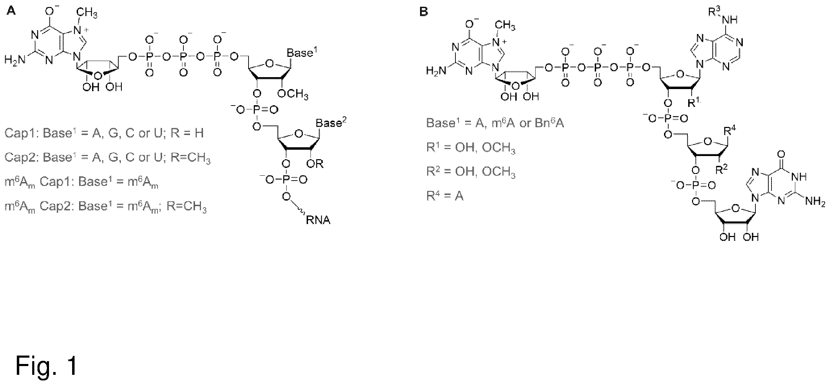

Linear mRNAs designed for therapeutic applications must be sequence-optimized and equipped with appropriate stabilizing elements at both ends: a 5' cap structure and a 3' polyA tail. The mRNA cap consists of an "inverted" 7-methylguanosine linked to the mRNA body by a 5'-5' triphosphate bridge, forming the so-called cap 0. This cap structure can be further modified by additional epitranscriptomic marks: a 2'-O methyl group on the ribose of the first transcribed nucleotide to form cap 1, on the first two nucleotides to form cap 2, or an N6-methyl group on the 5' terminal adenosine to form an m6Am cap, as illustrated in FIG 1.

The biological effects of epitranscriptomic modifications can be leveraged to improve the efficacy of mRNA as a therapeutic agent. In humans, cap 1 and cap 2 are crucial for distinguishing self from non-self RNA, helping to evade innate immune responses mediated by IFIT1. The role of the m⁶Am cap is still under debate but is likely related to mRNA senescence and evasion of innate immune responses. Notably, N6-methylation of 2'-O-methyladenosine in the cap (to form m⁶Am) is common epigenetic modification in all types of mammalian cells and is relatively abundant in several tissues. For example, in mice, the fraction of N6-methylated 5' terminal A reaches approximately 70% in the liver, 90% in the heart, and 94% in the brain. It has also been shown that m⁶Am increases the translational activity of mRNA in certain experimental setups (Sikorski et al., Nucleic Acids Research, Vol. 48 (4), 2020, 1607-1626).

The first commonly used method involves synthesizing in vitro transcribed mRNA with a cap 0 structure. This can be achieved using a dinucleotide cap analog or a Vaccinia Capping Enzyme (VCE). Following this, the 2'-O-methyl group is added to the first transcribed nucleotide using a 2'-O-methyltransferase. While this approach allows for the introduction of the cap 1 structure posttranscriptionally, it is often inefficient and labor-intensive. The additional enzymatic step increases the complexity and cost of the process, and achieving consistent high efficiency remains challenging.

The second method involves the co-transcriptional introduction of a trinucleotide primer that comprises the cap 1 structure. In this strategy, RNA is synthesized by T7 RNA polymerase using trinucleotide cap analog primers, such as those falling under the general formula m⁷GpppN¹ₘpN², ribonucleotide 5'-triphosphates (NTPs, such as ATP, CTP, GTP, and UTP, or their analogs like N1-methylpseudouridine or 5'-methoxyuridine), and a double-stranded DNA (dsDNA) template that contains a transcription promoter followed by a transcription initiation site. This site is characterized by the first nucleobase where the transcription actually starts (+1 position, denoted transcription start site (TSS)), whereas the sequence additionally including nucleobase positions +2 and optionally +3 following the TSS may be referred to as the initiating sequence (+1, +2), in certain embodiments a trinucleotide initiating sequence (TIS; +1, +2, +3). The formation of a transcriptionally active molecular complex depends on the specific promoter sequence and the polymerase, optionally including the hybridization of a capping primer in certain IVT reactions to the first transcribed nucleobases.

During transcription initiation in an IVT reaction, a molecular complex forms between the RNA polymerase, the non-coding (antisense) strand of the unwound region of the dsDNA template, and the cap analog. Nucleotides N¹ and N² of the primer hybridize (fully or partially complementarily) to the non-coding strand of the dsDNA template at or near the trinucleotide initiating sequence +1 and +2 positions **(****FIG 1****)** of the template, where the +1 position is defined as the predominant TSS in the absence of a cap analog (i.e., when producing uncapped RNA).

However, the effectiveness of this method varies widely. Ishikawa et al. demonstrated that m⁷GpppAₘpG can be used as a transcription primer to produce capped mRNA with a specific DNA template containing a <t>6.5 promoter followed by a GGG trinucleotide initiating sequence (TIS). In this case, the primer hybridized to the -1 and +1 positions of the dsDNA template (Ishikawa et al., Preparation of eukaryotic mRNA having differently methylated adenosine at the 5'-terminus and the effect of the methyl group in translation, Nucleic Acids Symposium Series No. 53 (2009), p. 129-130). Disadvantageously, this method required using a greater than six-fold excess of the capped initiating oligonucleotide primer, the most expensive nucleotide component of the transcription reaction, over competing GTP to drive the transcription reaction toward capped mRNA rather than uncapped mRNA. This significantly increases the total cost of synthesizing RNA, making the method economically impractical for large-scale production.

In WO2017053297A1, it was demonstrated that using a specific DNA template, which includes a <t>6.5 promoter followed by an AGG sequence, results in higher capping efficiencies for m⁷GpppAₘpG and similar compounds. Thus, it was concluded that ensuring complete complementarity of N¹ and N² to the +1 and +2 positions in the DNA template is crucial for achieving high capping efficiency of the final mRNA product. Additionally, it was claimed that for tetranucleotide cap analogs (such as m⁷GpppN¹ₘpN²ₘpN³), the best results are obtained when there is perfect complementarity between N¹, N², and N³ and the +1, +2, and +3 positions of the DNA template. This finding aligns with the prevailing and compelling belief reflected in the prior art that perfect alignment between the trinucleotide initiating sequence and the respective cap nucleotides is an essential prerequisite for efficient mRNA capping.

However, even with optimal reaction conditions, modifications designed to improve in vitro transcribed mRNA properties, such as the introduction of unnatural chemical groups within the 7-methylguanosine, triphosphate chain, the first phosphodiester bond, and N¹ nucleoside, have been used in this strategy, often resulted in decreased capping efficiency and overall transcription yield. For example, it was shown that while a benzyl group at the N6-position of 2'-O-methyladenosine may increase its translational activity, it significantly reduces capping efficiency and IVT yield. Achieving acceptable capping efficiency (>90%) for this analog required optimization of transcription conditions, including pH of the buffer, concentration of Mg²⁺ ions, and using a relatively high concentration of the cap analog, thereby limiting the application scope and increasing the overall cost of mRNA manufacturing (Warminski et al., Trinucleotide mRNA Cap Analog N6-Benzylated at the Site of Posttranscriptional m6Am Mark Facilitates mRNA Purification and Confers Superior Translational Properties In Vitro and In Vivo, JACS (2024), 146, 12, 8149-8163).

Furthermore, approximately 50% of eukaryotic mRNAs contain a cap 2 structure, which aids in evading the innate immune system and potentially enhances the efficacy of exogenously delivered mRNAs. mRNAs containing cap 2 can be produced in principle via IVT using tetrameric capped oligonucleotide primers (m⁷GpppN¹mpN²mpN³) as initiators for T7 RNA polymerase (Drazkowska et al., 2'-O-Methylation of the second transcribed nucleotide within the mRNA 5' cap impacts the protein production level in a cell-specific manner and contributes to RNA immune evasion, Nucleic Acids Research (2022), 50, 16, 9051-9071). However, there is currently no effective method for incorporating cap 2 into mRNA. Even with an optimal dsDNA template fully complementary at positions +1, +2, and +3, only about 50% capping efficiency is achieved (WO2017053297A1). A higher capping efficiency in the final mRNA product, crucial for most biotechnological and therapeutic applications, requires additional costly and time-consuming processing to remove immunogenic 5'-triphosphate RNA. This greatly increases both the cost and time required, which severely limits the economic feasibility of producing therapeutic mRNAs on an industrial scale and would likely fail to meet the prospective high demand for rapidly produced mRNA therapeutics.

Therefore, developing mRNAs with highly modified cap structures at their 5' ends, such as m⁷GpppBn⁶AₘpN, m⁷Gpppm⁶AₘpN, or cap 2 and its modified analogs, which are easily accessible and free from uncapped RNA impurities, is highly desirable. These mRNAs hold significant potential for therapeutic applications.

So far, it has never been systematically studied whether certain cap structures (tri- and tetranucleotide cap structures) and specific promoters, i.e., DNA templates, sequences are best compatible with achieving reproducibly high capping efficiency along with a favourably high translation rate.

As highly modified artificial cap structures are intricately dependent on the complex interplay between the structure of the DNA template, the trinucleotide initiating sequence, the cap analog, and the specific modifications of the cap analog, and as there is currently no general technical solution to achieve highly efficient IVT by using cap 2 primers, let alone efficiently incorporating a more heavily modified mRNA cap for enhanced translation and immune tolerance, there is consequently a great need in providing suitable cap primer:DNA template complexes that will reliably work in a setting using a specific promoter of interest and a cognate RNA polymerase to define the most cost efficient and save cognate primer::DNA template pair for a given setting.

### Summary of the Invention

The above-identified objectives are solved by the technical teaching as provided with the present invention.

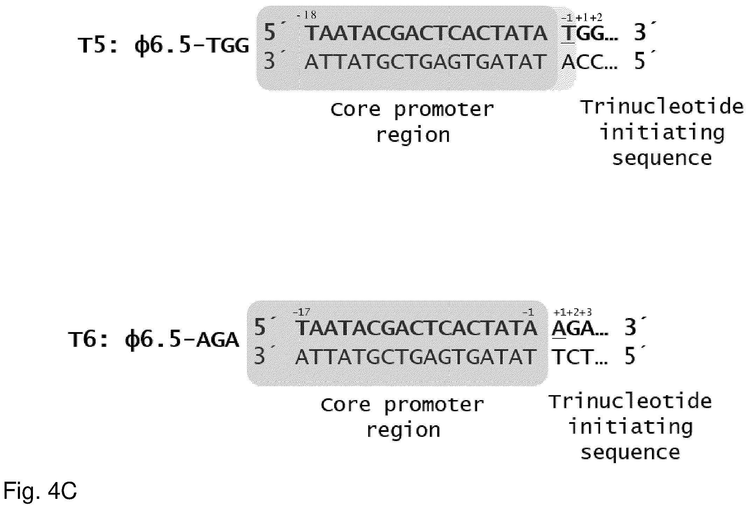

In a first aspect there is provided a molecular complex suitable for in vitro transcription with a T7 RNA polymerase comprising (i) a Cap analog according to the general formula (I) 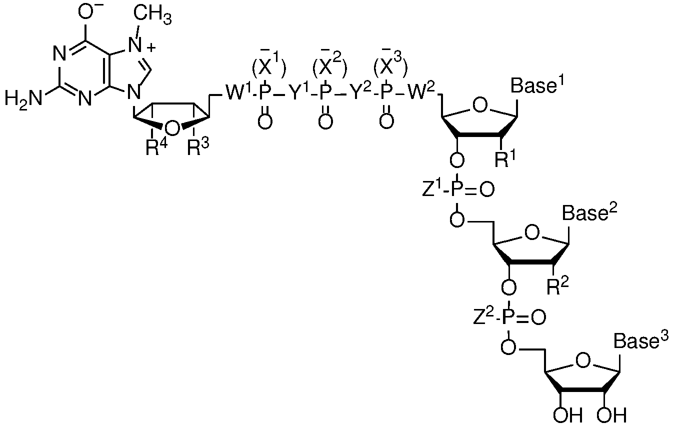 (I), wherein Base¹, Base² and Base³ are independently a natural, unnatural or modified base; R¹, R², R³ and R⁴ are independently OH, O-Me, H, F, Cl, O-alkyl, O-aryl, O-arylalkyl, O-acyl; wherein at least one from R¹ and R², or both, is/are not OH; and X¹, X², X³ are independently O, S, BH₃, Se; Y¹ and Y² are independently O, CH₂, CHCl, CHF, CF₂, CCl₂, NH; W¹ and W² are independently O, S; Z¹, Z² are independently O-, S-, CH₃, BH₃⁻ (ii) a DNA template comprising a T7 polymerase promoter and a trinucleotide initiating sequence (TIS); optionally: wherein nucleobase Base¹ hybridizes to the -1 position and/or preferably wherein nucleobase Base² hybridizes to the +1 position and Base³ hybridizes to the +2 position of the trinucleotide initiating sequence; or optionally: provided the DNA template comprises a core promoter of SEQ ID NO: 3 or the promoter comprises a a sequence of SEQ ID NOs: 12 or 13, nucleobase Base¹ hybridizes to the -2 position; nucleobase Base² hybridizes to the -1 position and Base³ hybridizes to the +1 position of the trinucleotide initiating sequence. In this embodiment the TIS may be a shortened sequence, starting with G at +1 and G at +2 (see FIG 4C).

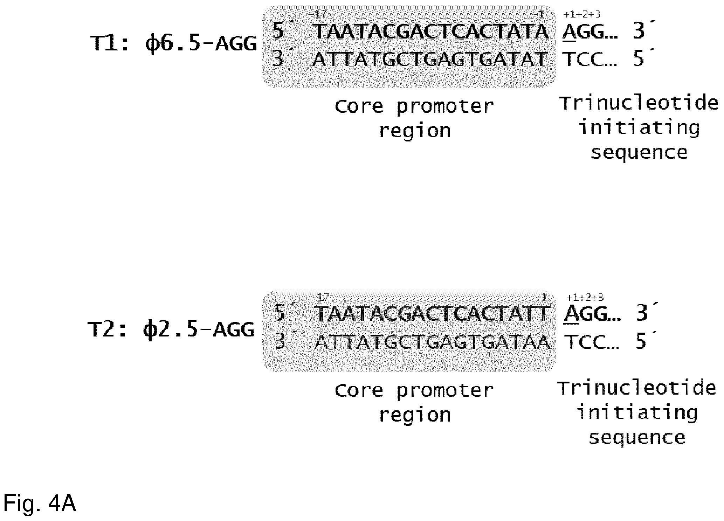

One embodiment relates to the molecular complex according to the first aspect, wherein the DNA template is selected from a T1 to T8 template as detailed in FIG 4A to 4D, or wherein the DNA template comprises a sequence as defined by any of SEQ ID NOs: 1 to 19, and/or the complement thereof, or a sequence having at least 99% identity to the respective sequence.

One embodiment relates to the molecular complex according to the first aspect or the previous embodiment thereof, wherein Base¹ and Base² of the Cap analog as defined in the first aspect (i) are independently selected from adenine or an analog of adenine, Base³ is guanine or an analog of guanine, and wherein the DNA template as defined in the first aspect (ii) comprises a promoter with a core region with a sequence as defined by any one of SEQ ID NO: 1 to 3, is selected from an AGG, an AAG, an ATG, a GGG, a GG, an AGA or a GAG.

One embodiment relates to the molecular complex according to the first aspect or any embodiment thereof, wherein Base¹ is independently adenine, N6-benzyladenine, N6-methyladenine, N6-(2-phenylethyl)adenine, and R1 and/or R2 are independently O-Me, and (i) Base² and Base³ are both guanine, or (ii) Base² is adenine and Base³ is guanine, or (iii) Base² and Base³ are independently guanine or adenine, or (iv) Base² is adenine and Base³ is uracil, or (v) Base² is uracil and Base³ is guanine in the Cap analog as defined in the first aspect or any embodiment thereof.

One embodiment relates to the molecular complex according to the first aspect or any embodiment thereof, wherein (i) Base¹ is ^{Bn6}Aₘ, Base² and Base³ are independently guanine, or (ii) Base¹ is ^{m6}Aₘ, Base² and Base³ are independently guanine, or (iii) Base¹ is ^{Bn6}Aₘ, Base² is adenine and Base³ is guanine, or (iv) Base¹ is ^{m6}Aₘ, Base² is adenine and Base³ is guanine, or (v) Base¹ is Aₘ, Base² is Gₘ, and Base³ is guanine, or (vi) Base¹ is ^{Bn6}Aₘ, Base² is Gₘ, and Base³ is guanine, or (vii) Base¹ is ^{m6}Aₘ, Base² is Gₘ, and Base³ is guanine, or (viii) Base¹ is Aₘ, Base² is Aₘ, and Base³ is guanine, or (ix) Base¹ is Aₘ, Base² is adenine, and Base³ is guanine, or (x) Base¹ is ^{Bn6}Aₘ, Base² is Aₘ, and Base³ is guanine, or (xi) Base¹ is ^{m6}Aₘ, Base² is Aₘ, and Base³ is guanine, or (xii) Base¹ is Aₘ, Base² and Base³ are both guanine, (xiii) Base¹ is N6-(2-phenylethyl)adenine, R1 is O-Me, preferably Base² and Base³ are independently guanine, in the Cap analog as defined in claim 1.

One embodiment relates to the molecular complex according to the first aspect or any embodiment thereof, additionally comprising a T7 RNA polymerase and/or a homolog thereof and/or a modified variant thereof.

A second aspect relates to a vector, or more than one vector, wherein the at least one vector comprises the at least one DNA template as defined in the first aspect or any embodiment thereof.

A third aspect relates to a DNA molecule, or more than one than one DNA molecule, wherein the at least one DNA molecule comprises the at least one DNA template as defined in the first aspect or any embodiment thereof.

A fourth aspect relates to a use of the molecular complex according to the first aspect or any embodiment thereof for in vitro transcription, wherein the capping efficiency is at least 80%, 81%, 82%, 83%, 84%, 85%, 86%, 87%, 88%, 89%, 90%, 91%, 92%, 93%, 94%, 95%, 96%, 97%, 98%, 99%, or up to 100%.

A fifth aspect relates to a method of producing at least one m⁷G-capped mRNA molecule, the method comprising: (i) providing a reaction mixture comprising the Cap analog as defined in the first aspect or any embodiment thereof, the DNA template as defined in the first aspect or any embodiment thereof and/or the vector according to the second aspect and/or the DNA molecule according to the third aspect and at least one T7 RNA polymerase and/or the sequence encoding the same; (ii) allowing in vitro transcription from said DNA template and/or from said vector and obtaining at least one m⁷G-capped mRNA; (iv) optionally: purifying said at least one m⁷G-capped mRNA; and/or (v) optionally: formulating said at least one m⁷G-capped mRNA molecule yielding a pharmaceutical composition.

One embodiment relates to the method of the fifth aspect further comprising a step of: (vii) introducing or contacting said at least one m⁷G-capped mRNA molecule obtained in step (iii) into or with at least one target cell in an in vitro cellular system for transfecting said at least one target cell and/or for in vitro studying the functionality of said at least one m⁷G-capped mRNA.

A sixth aspect relates to a pharmaceutical composition for use in a method of preventing and/or treating a disease, the pharmaceutical composition comprising the at least one m⁷G-capped mRNA molecule obtained or obtainable by the method according to the fifth aspect or the embodiment thereof, additionally comprising at least one pharmaceutically acceptable carrier or excipient.

One embodiment relates to the pharmaceutical composition for use in a method of preventing and/or treating a disease according to the sixth aspect, the pharmaceutical composition additionally comprising at least one lipid nanoparticle, wherein the at least one lipid nanoparticle is selected from the group of at least one liposome (LPs), at least one liposome-like nanoparticle (LLP), at least one solid lipid nanoparticle (SLN), at least one nanostructured lipid carrier (NLC), at least one lipid-polymer hybrid nanoparticle (LPN), at least one lipoprotein particle (LPT), at least one nanoemulsion, at least one cationic nanoemulsion (CNE) and at least one exosome.

A seventh aspect relates to a kit comprising the Cap analog as defined in the first aspect or any embodiment thereof, optionally comprising the DNA template as defined in the first aspect or any embodiment thereof, and/or the vector according to the second aspect and/or the DNA molecule according to the third aspect, optionally wherein the kit comprises at least one further reagent and/or at least one aqueous solution, preferably a buffer.

### Brief Description of the Figures

The following detailed description of the embodiments of the invention will be better understood when read in conjunction with the appended drawings. For the purpose of illustrating the invention, shown in the drawings are embodiments, which are presently exemplified. It should be understood, however, that the invention is not limited to the precise arrangement and instrumentalities of the embodiments shown in the drawings.

In all Figures, the lanes of the gels correspond to the numbers and samples summarized in **Table 3** and **Tables 5-9.**
**Figure 1 (FIG 1****):** Chemical structures of eukaryotic cap structures with different methylation patterns (A) and the general structure of synthetic cap analogs used as transcription initiation primers (B).

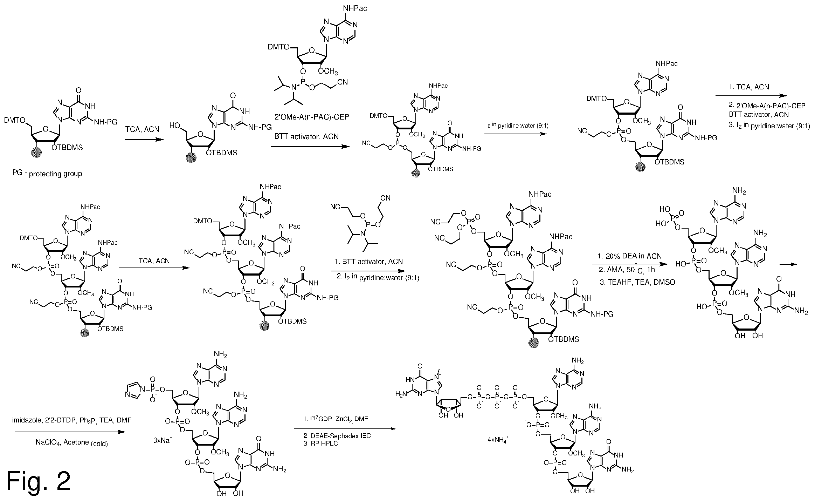

**Figure 2 (FIG 2****):** Synthesis of a representative primer carrying the cap 2 structure, m⁷GpppAₘpAₘpG. Other primers of similar structures (but different sequences) were/can be prepared using similar pathways using different commercially available phosphoramidites.

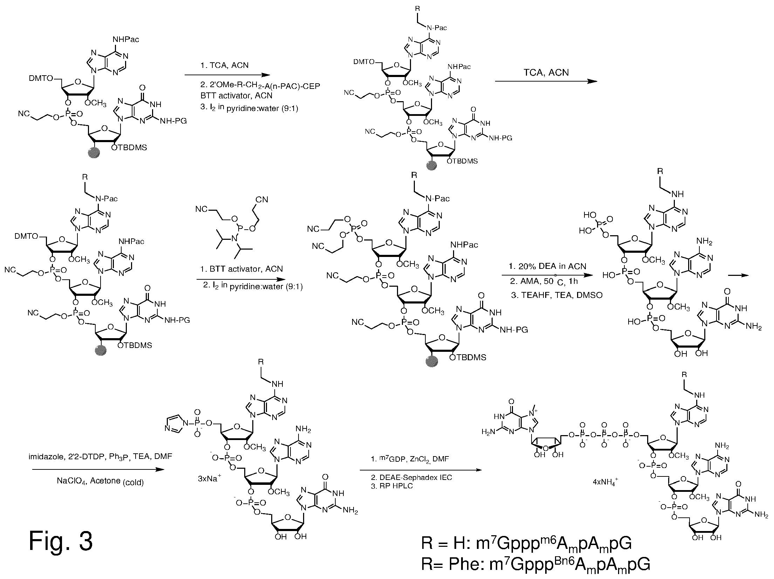

**Figure 3 (FIG 3****):** Synthesis of representative primers carrying cap 1 or cap 2 structures along with nucleobase modifications. Other primers with similar structures (but different sequences and nucleobase modifications) were/can be prepared using similar pathways using synthetic and commercially available phosphoramidites.

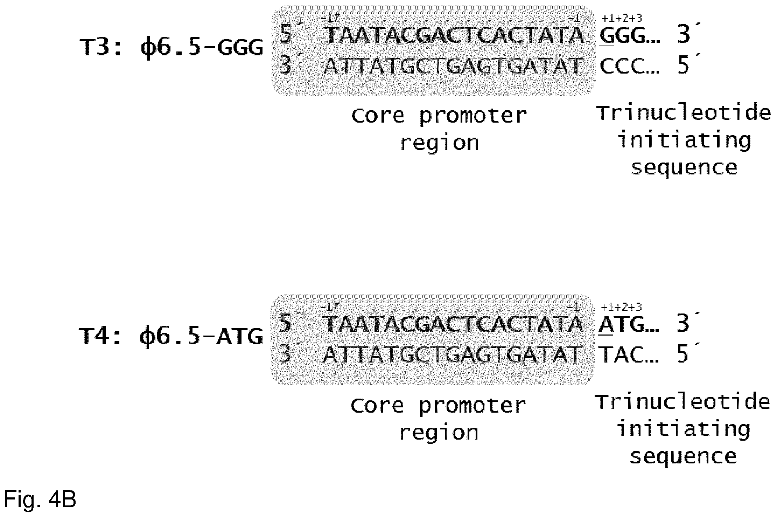

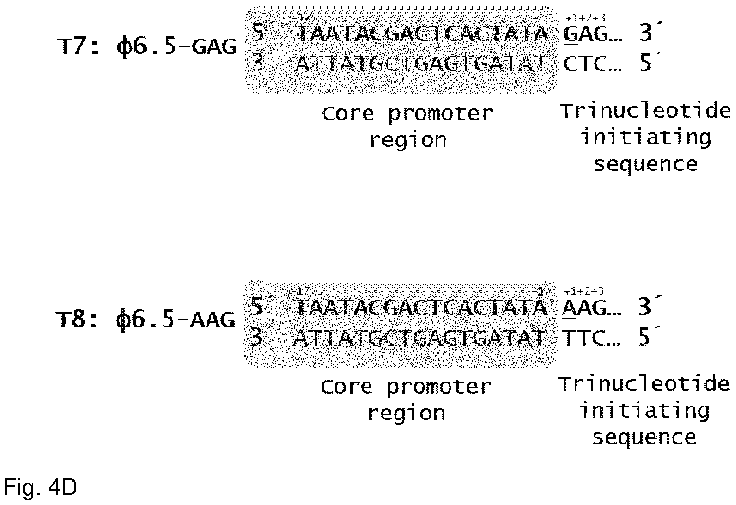

**Figure 4 (FIG 4A to D****):** Sequences of the promoters for T7 RNA polymerase and transcription start sites (TSSs) used in the experiments. Underlined nucleotide indicates the first transcribed nucleotide (+1 position; TSS), corresponding to the major initiation sites when uncapped mRNA synthesis is performed (i.e. in the absence of cap analog) (see also **Table 4).**

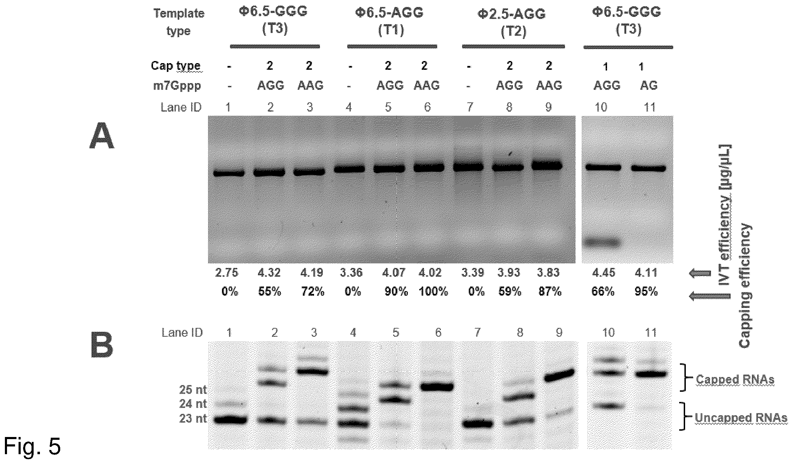

**Figure 5 (FIG 5****):** Results of in vitro transcription (IVT) with T7 RNA polymerase, in the presence of m⁷GpppAₘpGₘpG, m⁷GpppAₘpAₘpG (cap type 2 means 2'-O-methylated ribose of the nucleotide N¹ and N²) or without tetranucleotide cap analog, and the DNA templates T1 (Φ6.5-AGG), T2 (Φ2.5-AGG), and T3 (<t>6.5-GGG) (see also **Table 4).** IVT reactions were conducted for 2 h at 37°C in the presence of Bis-Tris buffer pH 6.5 and 25 mM MgCl₂, 5 mM ATP, CTP and UTP, 4 mM GTP, 10 mM tetranucleotide primer (for reactions providing uncapped mRNAs, cap analog was omitted), 40 ng/µL of linearized plasmid as the DNA template and T7 RNA polymerase (Roche). **A:** Analysis of the IVT efficiency of the crude transcription mixtures. For a rough comparison of the IVT efficiency the same amount (0.1 µL) of each crude IVT mix was applied on the agarose gel. Actual IVT efficiencies (values shown below the gel) were estimated after mRNA purification on the oligo(dT)₂₅ resin by measuring eluate volume and the absorbance at 260 nm. IVT efficiency was calculated as mRNA yield obtained from 1 µL of starting IVT volume. **B:** Capping efficiency analysis on the denaturing polyacrylamide gel for purified mRNAs cleaved with 5' UTR-specific ribozyme. Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gel).

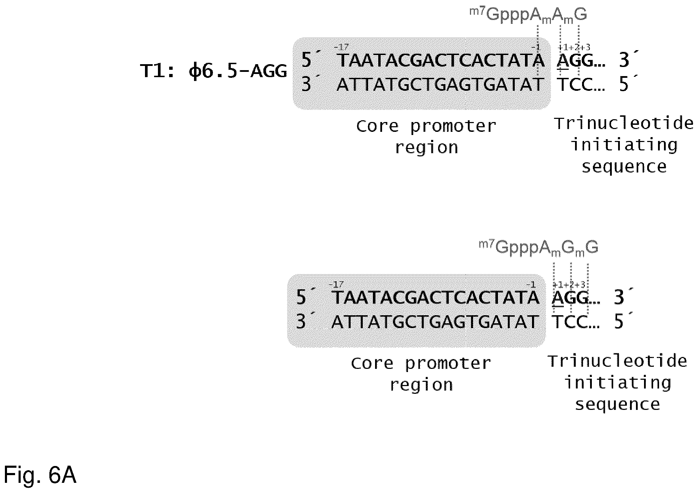

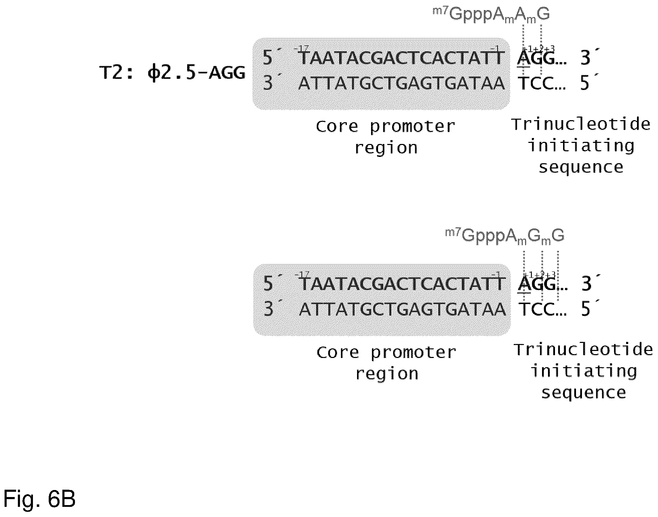

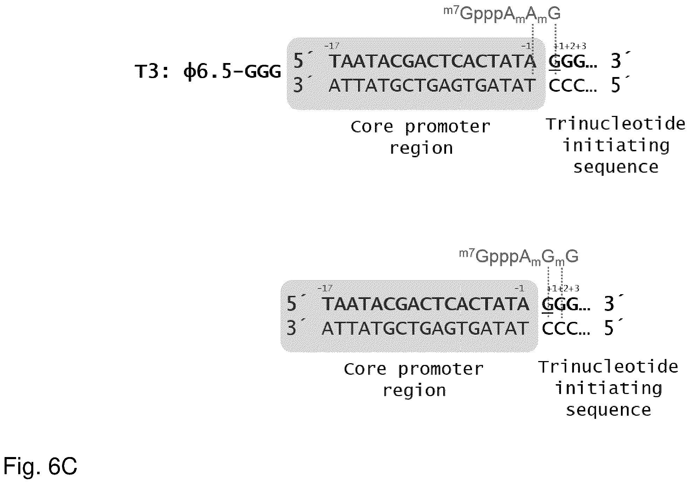

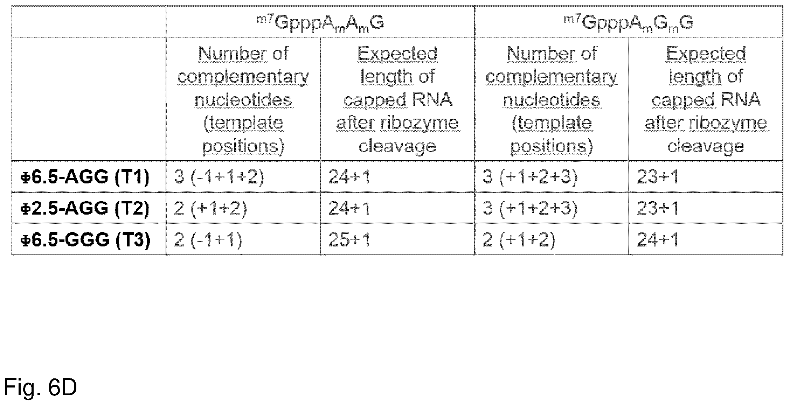

**Figure 6 (FIG 6A to D****):** The alignment of ^{m7}GpppA*pA*pG-type and ^{m7}GpppA*pG*pG-type tetranucleotide cap analogs with DNA templates T1 (Φ6.5-AGG) **(****FIG 6A****),** T2 (Φ2.5-AGG) **(****FIG 6B****)** or T3 (Φ6.5-GGG) **(****FIG 6C****)** during the major transcription initiation events (schemes on the upper panel). The table in **FIG 6D** summarizes expected lengths of the capped 5' end of RNA after ribozyme-mediated cleavage. Also, for each cap analog the number of nucleotides complementary to the particular template (T1, T2 or T3; see also **Table 4)** and hybridized positions (+1 corresponds to the first transcribed nucleotide) are presented.

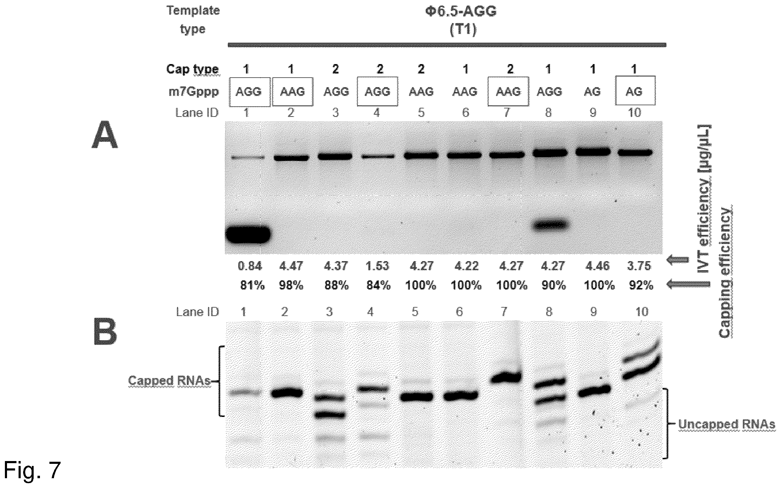

**Figure 7 (FIG 7****):** Results of in vitro transcription (IVT) with T7 RNA polymerase, in the presence of various tetranucleotide cap analog primers (10 mM each, and with Bn⁶-modified primers indicated with squares; see also **Table 3)** and template T1 (<t>6.5-AGG) (see also **Table 4)** comprising of Φ6.5-promoter and AGG transcription start site (i.e. with TTCC deoxyribonucleotides at positions -1, +1, +2 and +3 of the non-coding strands of the DNA template; see also **FIG 4****).** Trinucleotide cap analogs under their optimal conditions (described in **Example 3)** were used in control reactions (lanes 9 and 10). **A:** Analysis of the IVT efficiency of the crude transcription mixtures. The same amount of each IVT mix was applied on the 1×TBE 1.2% agarose gel, and each lane contains 0.1 µl of unpurified in vitro transcribed mRNA after DNase I treatment and 2× dilution with 50 mM EDTA (0.05 µl crude IVT per lane). Actual IVT efficiencies (values shown below the gel) were estimated after mRNA purification on the oligo(dT)₂₅ resin by measuring eluate volume and the absorbance at 260 nm. IVT efficiency was calculated as mRNA yield obtained from 1 µL of starting IVT volume. **B:** Capping efficiency analysis on the denaturing polyacrylamide gel for oligo(dT)₂₅-purified mRNAs cleaved with 5' UTR-specific ribozyme. The samples were run on a 15% polyacrylamide + 7 M urea gel in 1× TBE and stained with SYBR^{®} Gold (1:10,000) Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gel). Bands corresponding to ppp-RNA were identified by the experiment performed for RNA in vitro transcribed using template T1 (<t>6.5-AGG) and without cap analog added to the IVT mix (see **FIG 5B****).** All other conditions and NTP concentrations were as described in **Example 3.**

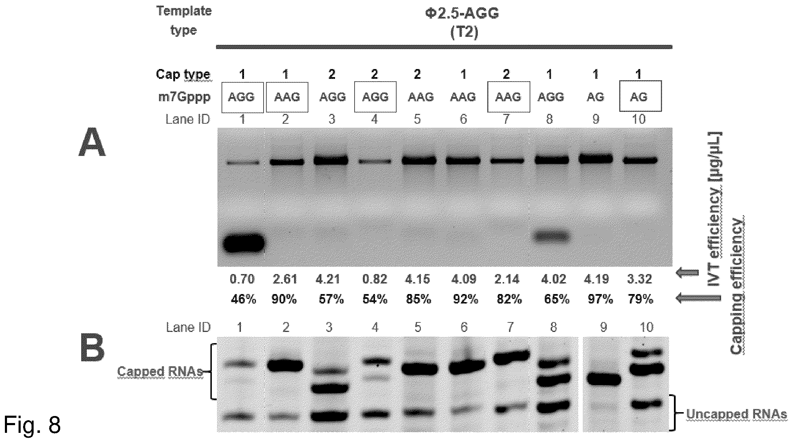

**Figure 8 (FIG 8****):** Results of in vitro transcription (IVT) with T7 RNA polymerase, in the presence of various tetranucleotide cap analog primers (10 mM each, and with Bn⁶-modified primers indicated with squares; see also **Table 3)** and template T2 (see also Table 4) comprising of Φ2.5-promoter and AGG transcription start site (i.e. with ATCC deoxyribonucleotides at positions -1, +1, +2 and +3 of the non-coding strands of the DNA template; see also **FIG 4A** to **D****).** Trinucleotide cap analogs under their optimal conditions (described in Example 3) were used in control reactions (lanes 9 and 10). **A:** Analysis of the IVT efficiency of the crude transcription mixtures. The same amount of each IVT mix was applied on the 1×TBE 1.2% agarose gel, and each lane contains 0.1 µl of unpurified in vitro transcribed mRNA after DNase I treatment and 2× dilution with 50 mM EDTA (0.05 µl crude IVT per lane). Actual IVT efficiencies (values shown below the gel) were estimated after mRNA purification on the oligo(dT)₂₅ resin by measuring eluate volume and the absorbance at 260 nm. IVT efficiency was calculated as mRNA yield obtained from 1 µL of starting IVT volume. **B:** Capping efficiency analysis on the denaturing polyacrylamide gel for oligo(dT)₂₅-purified mRNAs cleaved with 5' UTR-specific ribozyme. The samples were run on a 15% polyacrylamide + 7 M urea gel in 1× TBE and stained with SYBR^{®} Gold (1:10,000) Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gel). Bands corresponding to ppp-RNA were identified by the experiment performed for RNA in vitro transcribed using template T2 (Φ2.5-AGG) and without cap analog added to the IVT mix (see **FIG 5B****).** All other conditions and NTP concentrations were as described in **Example 3.**

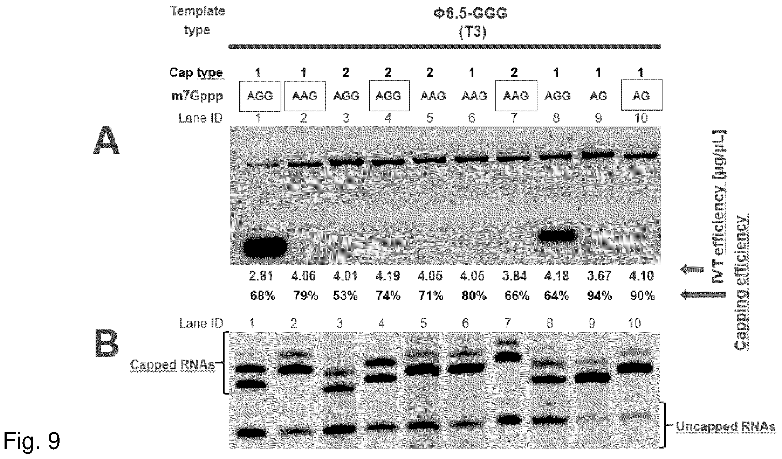

**Figure 9 (FIG 9****):** Results of in vitro transcription (IVT) with T7 RNA polymerase, in the presence of various tetranucleotide cap analog primers (10 mM each, and with Bn⁶-modified primers indicated with squares; see also **Table 3)** and template T3 (see also **Table 4)** comprising of Φ6.5-promoter and GGG transcription start site (i.e. with TCCC deoxyribonucleotides at positions -1, +1, +2 and +3 of the non-coding strands of the DNA template; see also **FIG 4****).** Trinucleotide cap analogs under their optimal conditions (described in **Example 3)** were used in control reactions (lanes 9 and 10). **A:** Analysis of the IVT efficiency of the crude transcription mixtures. The same amount of each IVT mix was applied on the 1×TBE 1.2% agarose gel, and each lane contains 0.1 µl of unpurified in vitro transcribed mRNA after DNase I treatment and 2× dilution with 50 mM EDTA (0.05 µl crude IVT per lane). Actual IVT efficiencies (values shown below the gel) were estimated after mRNA purification on the oligo(dT)₂₅ resin by measuring eluate volume and the absorbance at 260 nm. IVT efficiency was calculated as mRNA yield obtained from 1 µL of starting IVT volume. **B:** Capping efficiency analysis on the denaturing polyacrylamide gel for oligo(dT)₂₅-purified mRNAs cleaved with 5' UTR-specific ribozyme. The samples were run on a 15% polyacrylamide + 7 M urea gel in 1× TBE and stained with SYBR^{®} Gold (1:10,000) Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gel). Bands corresponding to ppp-RNA were identified by the experiment performed for RNA in vitro transcribed using template T3 (Φ6.5-GGG) and without cap analog added to the IVT mix (see **FIG 5B****).** All other conditions and NTP concentrations were as described in **Example 3.**

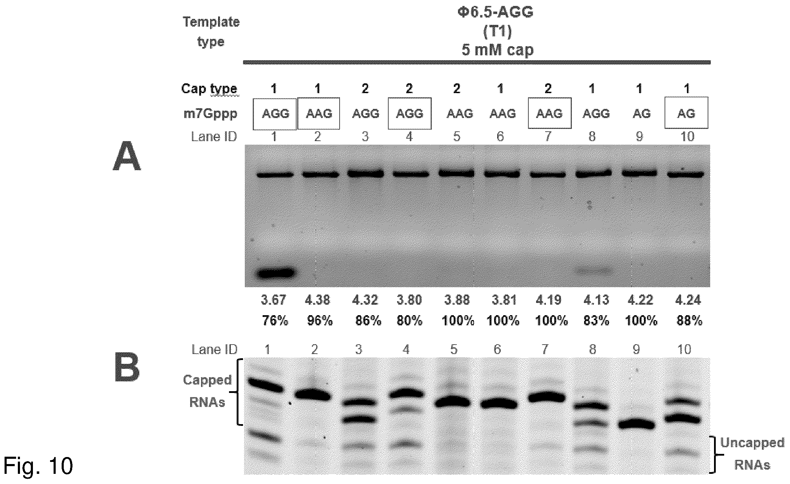

**Figure 10 (FIG 10****):** Efficient incorporation of 5 mM ^{m7}GpppA*pA*pG-type primers (Bn⁶-modified primers are indicated with squares; see also **Table 3)** by in vitro transcription (IVT) with template T1 (Φ6.5-AGG) (see also **Table 4).** IVT reactions were conducted for 2h at 37°C in the presence of Bis-Tris buffer pH 6.5 and 25 mM MgCl₂ (optimal for tetranucleotide transcription primers and Bn⁶-containing trinucleotide cap primer) or pH 8.0 and 10 mM MgCl₂ (optimal for trinucleotide cap 1 primer), 5 mM ATP, CTP and UTP, 4 mM GTP, 5 mM cap analog primer, 40 ng/µL of linearized plasmid as the DNA template and T7 RNA polymerase (Roche) (see also **Example 3). A:** Analysis of the IVT efficiency of the crude transcription mixtures. The same amount of each IVT mix was applied on the 1×TBE 1.2% agarose gel, and each lane contains 0.1 µl of unpurified in vitro transcribed mRNA after DNase I treatment and 2× dilution with 50 mM EDTA (0.05 µl crude IVT per lane). Actual IVT efficiencies (values shown below the gel) were estimated after mRNA purification on the oligo(dT)₂₅ resin by measuring eluate volume and the absorbance at 260 nm. IVT efficiency was calculated as mRNA yield obtained from 1 µL of starting IVT volume. **B:** Capping efficiency analysis on the denaturing polyacrylamide gel for oligo(dT)₂₅-purified mRNAs cleaved with 5' UTR-specific ribozyme. The samples were run on a 15% polyacrylamide + 7 M urea gel in 1× TBE and stained with SYBR^{®} Gold (1:10,000) Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gel). Bands corresponding to ppp-RNA were identified by the experiment performed for RNA in vitro transcribed using template T1 (Φ6.5-AGG) and without cap analog added to the IVT mix (see **FIG 5B****).** All other conditions and NTP concentrations were as described in **Example 3.**

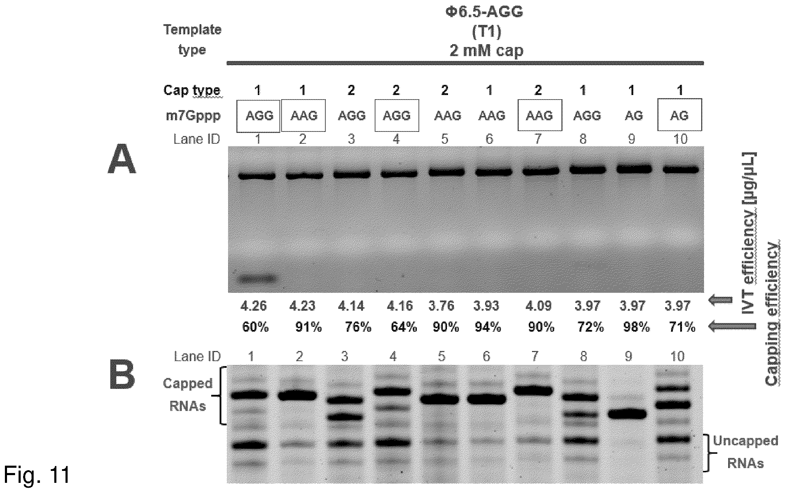

**Figure 11 (FIG 11****):** Efficient incorporation of 2 mM ^{m7}GpppA*pA*pG-type primers (Bn⁶-modified primers are indicated with squares; see also **Table 3)** by in vitro transcription (IVT) with template T1 (Φ6.5-AGG) (see also **Table 4).** IVT reactions were conducted for 2h at 37°C in the presence of Bis-Tris buffer pH 6.5 and 25 mM MgCl₂ (optimal for tetranucleotide transcription primers and Bn⁶-containing trinucleotide cap primer) or pH 8.0 and 10 mM MgCl₂ (optimal for trinucleotide cap 1 primer), 5 mM ATP, CTP and UTP, 4 mM GTP, 2 mM cap analog primer, 40 ng/µL of linearized plasmid as the DNA template and T7 RNA polymerase (Roche) (see also **Example 3). A:** Analysis of the IVT efficiency of the crude transcription mixtures. The same amount of each IVT mix was applied on the 1 ×TBE 1.2% agarose gel, and each lane contains 0.1 µl of unpurified in vitro transcribed mRNA after DNase I treatment and 2× dilution with 50 mM EDTA (0.05 µl crude IVT per lane). Actual IVT efficiencies (values shown below the gel) were estimated after mRNA purification on the oligo(dT)₂₅ resin by measuring eluate volume and the absorbance at 260 nm. IVT efficiency was calculated as mRNA yield obtained from 1 µL of starting IVT volume. **B:** Capping efficiency analysis on the denaturing polyacrylamide gel for oligo(dT)₂₅-purified mRNAs cleaved with 5' UTR-specific ribozyme. The samples were run on a 15% polyacrylamide + 7 M urea gel in 1× TBE and stained with SYBR^{®} Gold (1:10,000) Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gel). Bands corresponding to ppp-RNA were identified by the experiment performed for RNA in vitro transcribed using template T1 (Φ6.5-AGG) and without cap analog added to the IVT mix (see **FIG 5B****).** All other conditions and NTP concentrations were as described in **Example 3.**

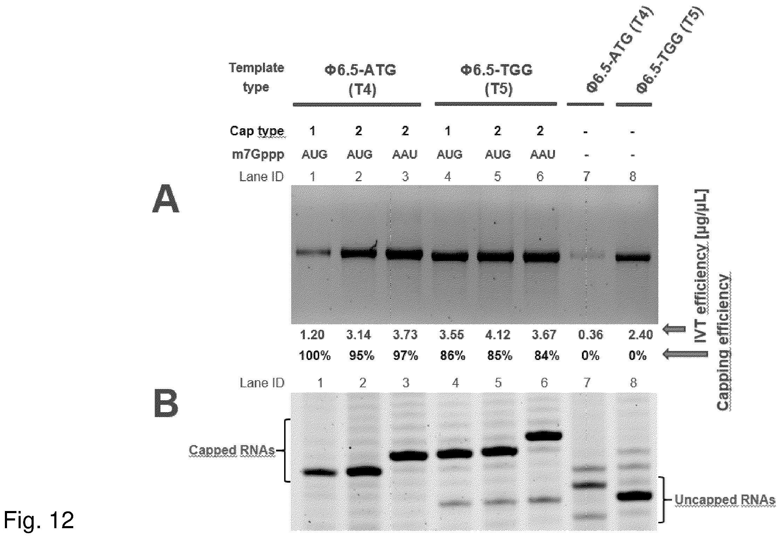

**Figure 12 (FIG 12****):** Incorporation of unmodified cap 1 and cap 2-type ^{m7}GpppA*pU*pG-primers and cap 2 m⁷GpppAₘpAₘpU into RNA by in vitro transcription (IVT) using template T4 (Φ6.5-ATG) and T5 (Φ6.5-TGG) (see also **Table 4)** in comparison to mRNA in vitro transcribed without cap primer. IVT reactions were conducted for 2h at 37°C in the presence of Bis-Tris buffer pH 6.5 and 30 mM MgCl₂, 5 mM ATP, CTP, UTP and GTP, 8 mM cap analog primer, 20 ng/µL of PCR-generated DNA template and T7 RNA polymerase (Roche) (see also **Example 4). A:** Analysis of the IVT efficiency of the crude transcription mixtures. The same amount of each IVT mix was applied on the 1×TBE 1.2% agarose gel, and each lane contains 0.1 µl of unpurified in vitro transcribed mRNA after DNase I treatment and 2× dilution with 50 mM EDTA (0.05 µl crude IVT per lane). Actual IVT efficiencies (values shown below the gel) were estimated after mRNA purification on the oligo(dT)₂₅ resin by measuring eluate volume and the absorbance at 260 nm. IVT efficiency was calculated as mRNA yield obtained from 1 µL of starting IVT volume. **B:** Capping efficiency analysis on the denaturing polyacrylamide gel for oligo(dT)₂₅-purified mRNAs cleaved with 5' UTR-specific ribozyme. The samples were run on a 15% polyacrylamide + 7 M urea gel in 1× TBE and stained with SYBR^{®} Gold (1 :10,000) Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gel). Bands corresponding to ppp-RNA were identified by the experiment performed for RNA in vitro transcribed using template T4 (Φ6.5-ATG) or T5 (Φ6.5-TGG) and without cap analog added to the IVT mix. All other conditions and NTP concentrations were as described in **Example 4.**

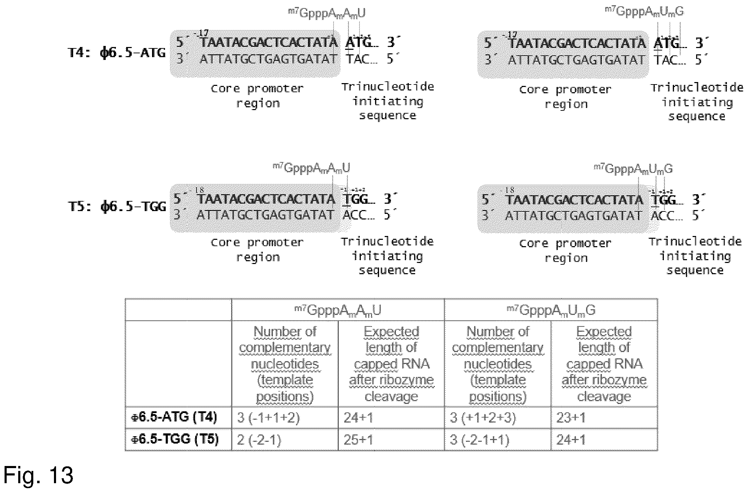

**Figure 13 (FIG 13****):** The alignment of m⁷GpppAₘpAₘpU and ^{m7}GpppA*pU*pG-type tetranucleotide cap analogs (^{m7}GpppAₘpUpG and ^{m7}GpppAₘpUₘpG) with DNA templates T4 (Φ6.5-ATG) or T5 (Φ6.5-TGG) during the major transcription initiation events (schemes on the upper panel). The table below summarizes expected lengths of the capped 5' end of RNA after ribozyme-mediated cleavage. Also, for each cap analog the number of nucleotides complementary to the particular template (T4 or T5; see also **Table 4)** and hybridized positions (+1 corresponds to the first transcribed nucleotide) are presented.

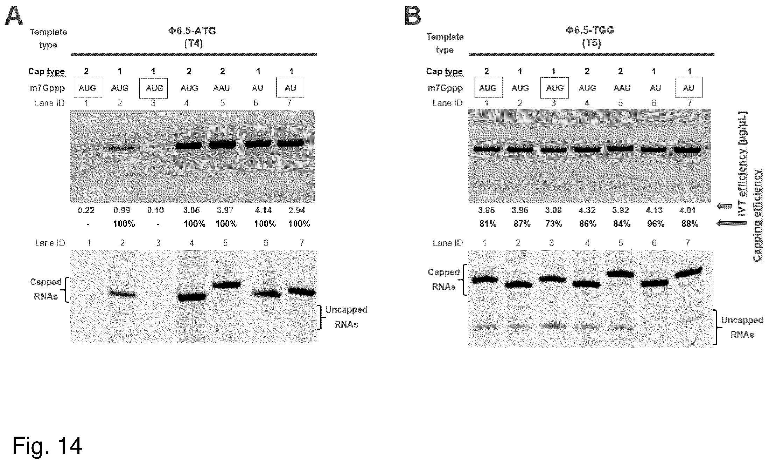

**Figure 14 (FIG 14****):** Incorporation of m⁷GpppAₘpAₘpU and ^{m7}GpppA*pU*pG-type primers into RNA by in vitro transcription (IVT) with A: template T4 (Φ6.5-ATG) and B: template T5 (<t>6.5-TGG) (see also **Table 4).** IVT reactions were conducted for 2h at 37°C in the presence of Bis-Tris buffer pH 6.5 and 30 mM MgCl₂ (optimal for ^{m7}GpppA*pU*pG-type tetranucleotide transcription primers and ^{m7}GpppA*pU-type trinucleotide primers), 5 mM ATP, CTP, UTP and GTP, 8 mM cap analog primer, 20 ng/µL of PCR-generated DNA template and T7 RNA polymerase (Roche) (see also **Example 4). Upper gels:** Analysis of the IVT efficiency of the crude transcription mixtures. The same amount of each IVT mix was applied on the 1×TBE 1.2% agarose gel, and each lane contains 0.1 µl of unpurified in vitro transcribed mRNA after DNase I treatment and 2× dilution with 50 mM EDTA (0.05 µl crude IVT per lane). Actual IVT efficiencies (values shown below the gels) were estimated after mRNA purification on the oligo(dT)₂₅ resin by measuring eluate volume and the absorbance at 260 nm. IVT efficiency was calculated as mRNA yield obtained from 1 µL of starting IVT volume. **Bottom gels:** Capping efficiency analysis on the denaturing polyacrylamide gel for oligo(dT)₂₅-purified mRNAs cleaved with 5' UTR-specific ribozyme. The samples were run on a 15% polyacrylamide + 7 M urea gel in 1× TBE and stained with SYBR^{®} Gold (1 :10,000) Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gels). Bands corresponding to ppp-RNA were identified by the experiment performed for RNA in vitro transcribed using template T4 (Φ6.5-ATG) or T5 (Φ6.5-TGG) and without cap analog added to the IVT mix (see **FIG 12****).** All other conditions and NTP concentrations were as described in **Example 4.**

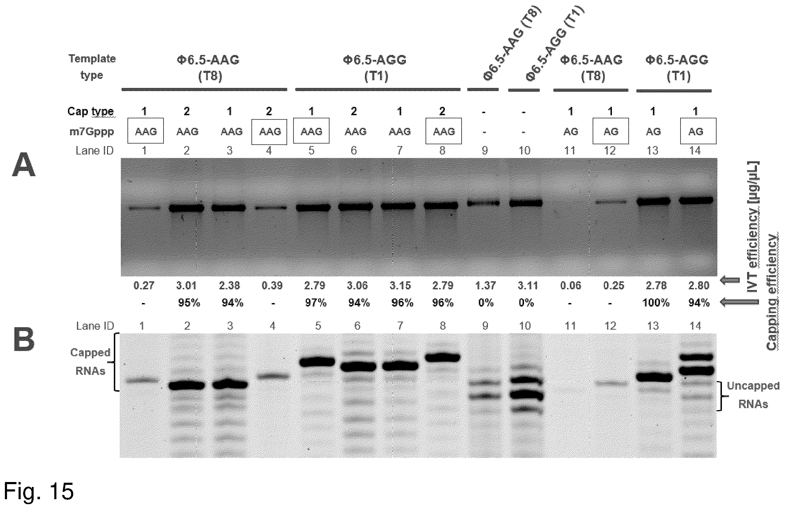

**Figure 15 (FIG 15****):** Incorporation of unmodified cap 1 and cap 2-type ^{m7}GpppA*pA*pG-primers and their Bn⁶-modified counterparts (Bn⁶-modified primers are indicated with squares) into RNA by in vitro transcription (IVT) with template T1 (Φ6.5-AGG; with complementarity of cap nucleotides N¹, N² and N³ with positions -1, +1 and +2 of the non-coding strand) and T8 (Φ6.5-AAG; with complementarity of cap nucleotides N¹, N² and N³ with positions +1, +2 and +3 of the non-coding strand) (see also **Table 4** and **FIG 6****).** Complementarity between positions -1, +1 and +2 and nucleotides N¹, N² and N³ of cap primer is essential for obtaining RNA with Bn6-modified 5' cap structure. **A:** Analysis of the IVT efficiency of the crude transcription mixtures. The same amount of each IVT mix was applied on the 1 ×TBE 1.2% agarose gel, and each lane contains 0.1 µl of unpurified in vitro transcribed mRNA after DNase I treatment and 2× dilution with 50 mM EDTA (0.05 µl crude IVT per lane). Actual IVT efficiencies (values shown below the gel) were estimated after mRNA purification on the oligo(dT)₂₅ resin by measuring eluate volume and the absorbance at 260 nm. IVT efficiency was calculated as mRNA yield obtained from 1 µL of starting IVT volume. **B:** Capping efficiency analysis on the denaturing polyacrylamide gel for oligo(dT)₂₅-purified mRNAs cleaved with 5' UTR-specific ribozyme. The samples were run on a 15% polyacrylamide + 7 M urea gel in 1× TBE and stained with SYBR^{®} Gold (1:10,000). Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gel). Bands corresponding to ppp-RNA were identified by the experiment performed for RNA in vitro transcribed using template T1 (Φ6.5-AGG) or T8 (Φ6.5-AAG) and without cap analog added to the IVT mix. All other conditions and NTP concentrations were as described in **Example 5.**

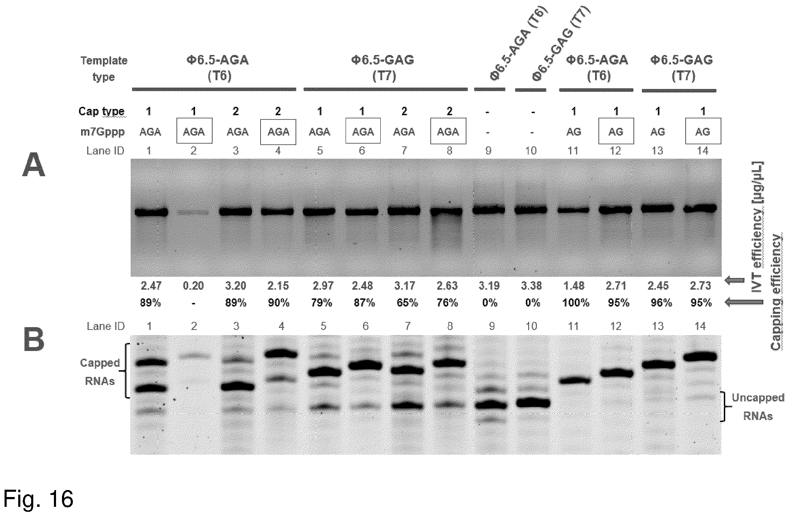

**Figure 16 (FIG 16****):** Incorporation of ^{m7}GpppA*pG*pA-type primers into RNA by in vitro transcription (IVT) with template T6 (Φ6.5-AGA; with complementarity of cap nucleotides N¹, N² and N³ with positions +1, +2 and +3 of the non-coding strand) and template T7 (Φ6.5-GAG; with complementarity of cap nucleotides N¹, N² and N³ with positions -1, +1 and +2 of the non-coding strand) (see also **Table 4)** (Bn⁶-modified primers are indicated with squares). **A:** Analysis of the IVT efficiency of the crude transcription mixtures. The same amount of each IVT mix was applied on the 1×TBE 1.2% agarose gel, and each lane contains 0.1 µl of unpurified in vitro transcribed mRNA after DNase I treatment and 2× dilution with 50 mM EDTA (0.05 µl crude IVT per lane). Actual IVT efficiencies (values shown below the gel) were estimated after mRNA purification on the oligo(dT)₂₅ resin by measuring eluate volume and the absorbance at 260 nm. IVT efficiency was calculated as mRNA yield obtained from 1 µL of starting IVT volume. **B:** Capping efficiency analysis on the denaturing polyacrylamide gel for oligo(dT)₂₅-purified mRNAs cleaved with 5' UTR-specific ribozyme. The samples were run on a 15% polyacrylamide + 7 M urea gel in 1× TBE and stained with SYBR^{®} Gold (1:10,000) Intensities of the visualized short capped and uncapped RNA (ppp-RNA) fragments were quantified densitometrically. For each sample capping efficiency was determined as the ratio of the measured intensity of the band (or bands) corresponding to capped RNA and the summarized intensities of capped RNA and ppp-RNA (percentage values shown above the gel). Bands corresponding to ppp-RNA were identified by the experiment performed for RNA in vitro transcribed using template T6 (Φ6.5-AGA) or T7 (Φ6.5-GAG) and without cap analog added to the IVT mix. All other conditions and NTP concentrations were as described in **Example 5.**

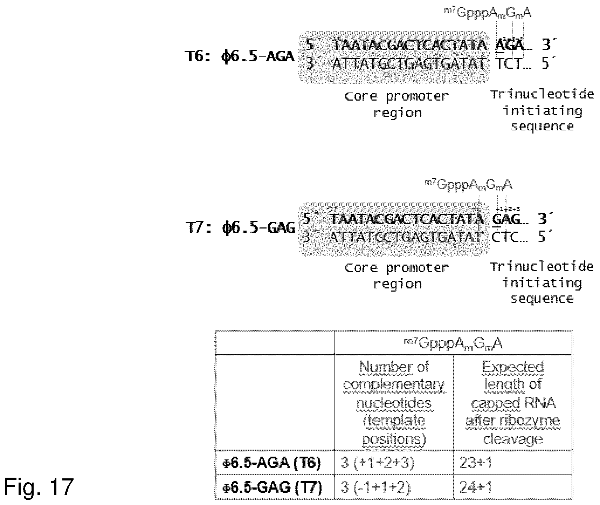

**Figure 17 (FIG 17****):** The alignment of ^{m7}GpppA*pG*pA-type tetranucleotide cap analogs (^{m7}GpppAₘpGpA, ^{m7}Gppp^{Bn6}AₘpGpA, ^{m7}GpppAₘpGₘpA, ^{m7}Gppp^{Bn6}AₘpGₘpA) with DNA template T6 (Φ6.5-AGA) or T7 (Φ6.5-GAG) during the major transcription initiation events (schemes on the upper panel). The table below summarizes expected lengths of the capped 5' end of RNA after ribozyme-mediated cleavage. Also, for each cap analog the number of nucleotides complementary to the particular template (T6 or T7; see also **Table 4)** and hybridized positions (+1 corresponds to the first transcribed nucleotide) are presented.

### Brief Description of Sequences

| **SEQ ID NO** | **Description** |
|---|---|
| 1 | Φ6.5 Core promoter region |
| 2 | Φ2.5 Core promoter region |
| 3 | Φ6.5 Core promoter region T extended |
| 4 | T1 Φ6.5-AGG upper strand **(****FIG 4****,** **FIG 6****)** |
| 5 | T1 Φ6.5-AGG lower strand **(****FIG 4****,** **FIG 6****)** |
| 6 | T2 Φ2.5-AGG upper strand **(****FIG 4****,** **FIG 6****)** |
| 7 | T2 Φ2.5-AGG lower strand **(****FIG 4****,** **FIG 6****)** |
| 8 | T3 Φ6.5-GGG upper strand **(****FIG 4****,** **FIG 6****)** |
| 9 | T3 Φ6.5-GGG lower strand **(****FIG 4****,** **FIG 6****)** |
| 10 | T4 Φ6.5-ATG upper strand **(****FIG 4****,** **FIG 13****)** |
| 11 | T4 Φ6.5-ATG lower strand **(****FIG 4****,** **FIG 13****)** |
| 12 | T5 Φ6.5-TGG upper strand **(****FIG 4****,** **FIG 13****)** |
| 13 | T5 Φ6.5-TGG lower strand **(****FIG 4****,** **FIG 13****)** |
| 14 | T6 Φ6.5-AGA upper strand **(****FIG 4****,** **FIG 17****)** |
| 15 | T6 Φ6.5-AGA lower strand **(****FIG 4****,** **FIG 17****)** |
| 16 | T7 Φ6.5-GAG upper strand **(****FIG 4****,** **FIG 13****,** **FIG 17****)** |
| 17 | T7 Φ6.5-GAG lower strand **(****FIG 4****,** FIG 13, **FIG 17****)** |
| 18 | T8 Φ6.5-AAG upper strand **(****FIG 4****)** |
| 19 | T8 Φ6.5-AAG lower strand **(****FIG 4****)** |

Notably, SEQ ID Nos: 4 and 5, 6 and 7, 8 and 9, 10 and 11, 12 and 13, 14 and 15, 16 and 17, and 18 and 19 each represent pairs forming a dsDNA template named T1 to T8, respectively, as shown in **FIG 4A** to **FIG 4D****.** As the skilled person understands that each strand (5' to 3'and 3'to 5' notation) in a pair of sequences is fully complementary to each other, reference to one of these sequences only will also be enough for the skilled person to identify a suitable dsDNA template selected from T1 to T8 based on the information related to one (single-stranded) sequence / SEQ ID NO only.

### Detailed Description

The present invention solves the problems in the prior art by providing molecular complexes that reliably initiate in vitro transcription catalysed by T7 RNA polymerase using highly modified caps, achieving remarkably higher capping efficiencies and IVT efficiencies than those deemed optimal in prior art. Contrary to the common belief, we discovered that tetranucleotide primers (m⁷GpppN¹ₘpN²ₘpN³) of specific sequences are most effectively incorporated when aligned with a dsDNA template that ensures nucleosides N² and N³ align and hybridize with the +1 and +2 positions of the template. These novel complexes enable the production of mRNAs with heavily modified cap structures (e.g., incorporating the ^{Bn6}Aₘ modification, cap 2, and their combinations) with capping efficiencies exceeding 90% and reaching up to 100%, without compromising the in vitro transcription yield. In addition, the present invention provides not only a solution to the problem of efficiently incorporating a tetrameric cap structure and its modified derivates into mRNA, but also enables more effective, cost sensitive and thus upscalable synthesis of mRNAs carrying a cap 1 structure.

To solve the prevailing challenges in the field of synthetic mRNA production, particularly the suboptimal incorporation of modified caps which are crucial for enhanced translation efficiency and reduced immune response, the present invention provides a surprising solution. Traditional methods have struggled with low capping efficiencies and incomplete cap incorporation, which severely limits the widescale application of therapeutic mRNA.

The present invention introduces a novel approach utilizing tetranucleotide primers that are aligned with a dsDNA template, ensuring that the nucleosides in positions N² and N³ of the primer hybridize to the +1 and +2 sites of the transcription template. This alignment is critical because it maximizes the efficiency with which cap analogs, particularly complex and modified structures such as the ^{Bn6}Aₘ modification and cap 2 analogs, are incorporated into the mRNA strand.

So far in the art, no detailed study is available analyzing the importance of the primer sequence for capping efficiency and IVT yield for different promoter structures / DNA templates even though knowledge about these molecular details is of outstanding importance to define versatile primer::DNA template pairs performing best for a given experiment *in vitro* and, particularly, *in vivo.* Additionally, the present invention for the first time provides a highly systematic comparison of tetranucleotide and comparative trinucleotide primers and certain DNA templates corresponding to naturally occurring promoter regions, or DNA templates derived and closely related to naturally occurring promoter regions to identify primer::DNA template complexes that synergistically work with each other by forming a sterically favouring complex that promotes both: transcription initiation and high translational activity. Thereby, novel and generally applicable complexes are provided that are suitable for various different IVT applications depending on the individual need.

A key advantage of this invention is its remarkable efficiency in cap incorporation - exceeding 90% and in some instances reaching 100%. Such high levels of efficiency are unprecedented in the prior art relating to IVT and represent a significant technical leap forward. Moreover, these high efficiencies are achieved without compromising the yield of the transcription process itself, which remains robust.

This method not only addresses the technical difficulties previously encountered with cap incorporation but also enhances the overall cost-effectiveness and scalability of mRNA synthesis. By enabling the reliable production of mRNAs with complex cap structures, this invention opens up new possibilities for the development of mRNA-based therapeutics, offering potential treatments for a wide range of diseases with improved efficacy and reduced side effects. The ability to consistently produce high-quality, capped mRNA in a cost-effective and scalable manner is particularly advantageous for rapid vaccine development and personalized medicine applications.

In a first aspect there is provided a molecular complex suitable for in vitro transcription with a T7 RNA polymerase comprising (i) a Cap analog according to the general formula (I) 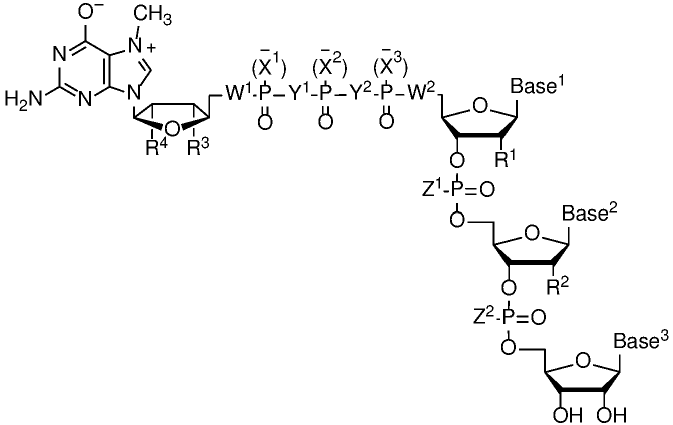 (I), wherein Base¹, Base² and Base³ are independently a natural, unnatural or modified base; R¹, R², R³ and R⁴ are independently OH, O-Me, H, F, Cl, O-alkyl, O-aryl, O-arylalkyl, O-acyl; wherein at least one from R¹ and R², or both, is/are not OH; and X¹, X², X³ are independently O, S, BH₃, Se; Y¹ and Y² are independently O, CH₂, CHCl, CHF, CF₂, CCl₂, NH; W¹ and W² are independently O, S; Z¹, Z² are independently O-, S-, CH₃, BH₃- (ii) a DNA template comprising a T7 polymerase promoter and a trinucleotide initiating sequence; optionally: wherein nucleobase Base¹ hybridizes to the -1 position and/or preferably wherein nucleobase Base² hybridizes to the +1 position and Base³ hybridizes to the +2 position of the trinucleotide initiating sequence; or optionally: provided the DNA template comprises a core promoter of SEQ ID NO: 3 or the promoter comprises a a sequence of SEQ ID NOs: 12 or 13, nucleobase Base¹ hybridizes to the -2 position; nucleobase Base² hybridizes to the -1 position and Base³ hybridizes to the +1 position of the trinucleotide initiating sequence.

The T7 polymerase promoter typically spans from nucleotide positions -18 or -17 to +6 relative to the transcription start site is particularly critical for the binding of T7 RNA polymerase. The T7 RNA polymerase binds to the promoter DNA with high specificity between positions -18 or -17 and -5. A "T7 polymerase promoter" as used herein refers to any naturally occurring or slightly amended (>98%, or >99% identity for the core promoter region spanning from about -18 to -1) T7 promoter that can be recognized by T7 RNA polymerase in the presence of a (natural or artificial) cap primer so that transcription is initiated. This region contains specific sequences that are recognized and bound by the polymerase to ensure correct positioning and orientation for transcription initiation. As part of the initiation process, the double-stranded DNA is melted from positions -4 to +3. This melting creates a single-stranded template necessary for the initiation of RNA synthesis. The melting of the DNA at this site allows the polymerase to access the template strand where RNA synthesis begins at the +1 position (Cheetham et al., Structural basis for initiation of transcription from an RNA polymerase-promoter complex. Nature (1999), 399, 80-83; Cheetham et al., Structure of a transcribing T7 RNA polymerase initiation complex. Science (1999), 286, 2305-2309). In the present invention, positions -17 to -1 for T1 to T4 and T6 to T8, or positions -18 to -1 for T5, respectively, relative to a transcription start site are denoted "core promoter region". Two preferred core promoter regions are defined by SEQ ID NOs: 1 and 2. The subsequent three nucleobases on positions +1, +2, +3 relative to a transcription start site are denoted as trinucleotide initiating sequence. The transcription start site is defined as the site were transcription is initiated by formation of a transcription complex comprising a promoter sequence and a polymerase. T1 to T8 each including the +1, +2, +3 relative to the TSS are represented in SEQ ID NOs: 3 to 18, respectively.

Natural nucleobases consist of the canonical purines-adenine (A) and guanine (G)-and pyrimidines - cytosine (C), thymine (T), and uracil (U) found in the nucleic acids DNA and RNA. These bases engage in specific Watson-Crick base pairing, where adenine pairs with thymine (in DNA) or uracil (in RNA) and cytosine pairs with guanine, facilitated by hydrogen bonds. These bases are designed e.g., to expand the genetic code by introducing new base pairs that are capable of stable and replicable interactions, yet distinct from the traditional base pairing systems. Unnatural bases may engage in alternative hydrogen bonding patterns or utilize other types of chemical interactions such as hydrophobic forces or metal coordination, thereby enabling the creation of novel nucleic acid architectures with enhanced or novel biochemical properties. Modified nucleobases are derivatives of natural nucleobases that have been chemically altered either post-synthetically or through enzymatic processes within cells. Modified nucleobases such as 5-Methylcytosine (5-mC), 5-hydroxymethylcytosine (5-hmC), N6-methyladenine (^{m6}A), N6-benzyladenine, (^{Bn6}A), N6-(2-phenylethyladenine) (^{Phet6}A), hypoxanthin, but not limited thereof, or the addition of complex groups alter the physicochemical properties of the bases. Such modifications can influence DNA stability, the regulation of gene expression, and the epigenetic landscape, and contribute to processes like DNA repair, replication, and cellular differentiation. Notably, modified nucleobases can be strategically utilized to harness specific biological effects, significantly enhancing the efficacy of mRNA therapeutics.

One embodiment relates to the molecular complex according to the present invention, wherein the DNA template comprises a sequence as defined by any of SEQ ID NOs: 1 to 19, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or preferably at least 99% sequence identity to any of the SEQ ID NOs: 1 to 189, respectively, wherein the sequence comprises a portion spanning positions -1 to -17 upstream of the initiating sequence (-1 to -18 relative to the TSS +1 site for the T5 DNA template sequences with SEQ ID NOs: 12 and 13, respectively).

One embodiment relates to the molecular complex according to the present invention, wherein Base¹ and Base² of the Cap analog as defined in the first aspect (i) are independently selected from adenine or an analog of adenine, Base³ is guanine or an analog of guanine, and wherein the DNA template (ii) comprises a promoter with a core region with a sequence as defined by any one of SEQ ID NO: 1 to 3, is selected from an AGG, an AAG, an ATG, a GGG, a GG, an AGA or a GAG.

For certain DNA templates, herein and in the literature, two Gs at the +1 and the +2 site of a promoter / DNA template may be rather difficult for IVT reactions, as this configuration may form some tertiary structures in the presence of metal cations. The present inventors thus systematically studied the influence and importance of the +1

In certain embodiments, the promoter is a phage-derived promoter, preferably, a Φ2.5-promoter, more preferably a Φ6.5-promoter. Preferable embodiments of these two promoter types are illustrated in **FIG 4****.**

In certain embodiments, the DNA template comprises at least one synthetic and/or at least one unnatural nucleoside.

Synthetic nucleoside analogs play vital roles in expanding the applications of DNA in research, diagnostics and therapy. Notable examples include BrdU (5-Bromo-2'-deoxyuridine) and EdU (5-ethynyl-2'-deoxyuridine). Locked Nucleic Acids (LNAs) and Phosphorothioate nucleotides introduce backbone modifications that enhance duplex stability and resistance to nucleases. Moreover, unnatural nucleosides like dNaM, dTPT3, NaM, TPT3, dMMO2^{Bio}, d5SICS dNaM have been developed to potentially expand the genetic code, enabling the creation of novel proteins and biomolecules in synthetic biology (Fischer et al., New codons for efficient production of unnatural proteins in a semisynthetic organism. Nat Chem Biol (2020), 16, 570-576).

One embodiment relates to the molecular complex according to the present invention, wherein Base¹ is independently adenine, N6-benzyladenine, N6-methyladenine, N6-(2-phenylethyl)adenine, and R1 and/or R2 are independently O-Me, and (i) Base² and Base³ are both guanine, or (ii) Base² is adenine and Base³ is guanine, or (iii) Base² and Base³ are independently guanine or adenine, or (iv) Base² is adenine and Base³ is uracil, or (v) Base² is uracil and Base³ is guanine in the Cap analog according to the present invention.

One embodiment relates to the molecular complex according to the present invention, wherein (i) Base¹ is ^{Bn6}Aₘ, Base² and Base³ are independently guanine, or (ii) Base¹ is ^{m6}Aₘ, Base² and Base³ are independently guanine, or (iii) Base¹ is ^{Bn6}Aₘ, Base² is adenine and Base³ is guanine, or (iv) Base¹ is ^{m6}Aₘ, Base² is adenine and Base³ is guanosine, or (v) Base¹ is Aₘ, Base² is Gₘ, and Base³ is guanine, or (vi) Base¹ is ^{Bn6}Aₘ, Base² is Gₘ, and Base³ is guanine, or (vii) Base¹ is ^{m6}Aₘ, Base² is Gₘ, and Base³ is guanine, or (viii) Base¹ is Aₘ, Base² is Aₘ, and Base³ is guanine, or (ix) Base¹ is Aₘ, Base² is adenine, and Base³ is guanine, or (x) Base¹ is ^{Bn6}Aₘ, Base² is Aₘ, and Base³ is guanine, or (xi) Base¹ is ^{m6}Aₘ, Base² is Aₘ, and Base³ is guanine, or (xii) Base¹ is Aₘ, Base² and Base³ are both guanine, (xiii) Base¹ is N6-(2-phenylethyl)adenine, R1 is O-Me, preferably Base² and Base³ are independently guanine, in the Cap analog as defined in claim 1.

In certain embodiments, the cap is ^{m7}GpppA*pA*pG, ^{m7}GpppA*pA*pU, ^{m7}GpppA*pA*pC or ^{m7}GpppA*pU*pG, each independently comprising a natural, unnatural or modified base, but not limited thereof.

Different types of benzyl derivatives added to the N6-position of 2'-O-methyladenosine may also be biologically active, comprising a para-substituted benzyl group, such as p-methylbenzyl, p-methoxybenzyl, p-nitrobenzyl, p-hydroxybenzyl, or p-fluorobenzyl, a meta-substituted benzyl group, such as m-methylbenzyl or m-chlorobenzyl, an ortho-substituted benzyl group, such as o-methylbenzyl, o-hydroxybenzyl, a di-substituted benzyl group, such as 2,4-dichlorobenzyl, or a heteroaromatic benzyl group, such as thiophenylmethyl or 2-pyridylmethyl, a benzyl homologue, such as homobenzyl (2-phenylethyl), 3-phenylpropyl, or arylalkyl derivatives such as α-napthylmethyl, β-naphtylmethyl, napthylethyl.

In certain embodiments, the N6-position can also be advantageously modified by an alkylaryl substituent comprising a fluorescent dye such as pyrene, coumarine, fluorescein, rhodamine or cyanine.

One embodiment relates to the molecular complex according to the present invention, additionally comprising a T7 RNA polymerase and/or a homolog thereof and/or a modified variant thereof.

T7 RNA polymerase is a highly specific enzyme from the T7 bacteriophage that is instrumental in transcribing DNA into RNA. It is part of a broader family of single-subunit RNA polymerases (RNAPs) that share structural and functional characteristics. This family includes other phage RNAPs like T3, T6, K11, SP6, and N4, which are similar to T7 RNA polymerase in terms of their mechanism of action and specificity for their respective phage DNA. Additionally, this family encompasses mitochondrial RNA polymerases, which are crucial for transcription within mitochondria.

Enzymes, including polymerases, can be artificially modified through several advanced techniques to enhance their properties, such as stability, activity, and specificity, suiting them for various applications in biotechnology and medicine. Traditional DNA polymerases, while efficient, often lack the ability to perform certain desired functions that new biotechnological applications demand. To address this, innovative engineering methods such as mutagenesis and the creation of protein chimeras have been employed. These methods have allowed scientists to expand the functionality of DNA polymerases beyond their natural capacities. For instance, engineered polymerases can now tolerate harsh conditions and the presence of inhibitors, and are capable of replicating DNA templates that contain chemically modified nucleotides. A key aspect of this engineering effort is the development of novel selection techniques, such as using water-in-oil emulsions that link genotype to phenotype more effectively than traditional methods like phage display. This approach has facilitated the exploration of an extended sequence space, leading to the discovery of polymerases that are more resilient and versatile. The continuous expansion of the "polymerase universe" is in step with the increasing chemical diversity of modified nucleotides, enabling the development of DNA polymerases with unnatural or enhanced functions that are pivotal for advancing biotechnological applications (Coulther el al., Engineering polymerases for new functions, Trends in Biotechnology (2019), 37, 10, 1091-1103).

A second aspect relates to a vector, or more than one vector, wherein the at least one vector comprises the at least one DNA template according to the present invention.

A vector designed for mRNA production is a DNA construct engineered to facilitate the synthesis of specific mRNA molecules. It comprises certain genetic elements, including a promoter, coding sequence, optionally a polyadenylation signal, and may incorporate other components like selection markers and origins of replication. A vector according to the present invention are based on plasmids. These plasmid backbones contain essential elements required for the replication and maintenance of the vector within the host cell.

A third aspect relates to a DNA molecule, or more than one than one DNA molecule, wherein the at least one DNA molecule comprises the at least one DNA template according to the present invention.

In the production of mRNA through IVT, various templates can be utilized aside from commonly used plasmid vectors, each offering unique advantages depending on the specific needs of the research or production process. PCR-generated templates are often used for rapid and cost-effective mRNA synthesis, involving a DNA fragment amplified by PCR that includes the appropriate promoter upstream of the gene of interest. This method circumvents the need for cloning, expediting the process. Alternatively, plasmid DNA can be linearized using restriction enzymes that cut downstream of the insert sequence to provide a clean and efficient template for IVT.

Synthetic gene blocks, which are short, double-stranded DNA molecules including a promoter sequence and the gene of interest, can be ordered from commercial suppliers and are ready to use without further preparation. Less commonly, single-stranded DNA (ssDNA) with the necessary promoter sequences can also serve as a template, though additional steps may be required to ensure stability and efficiency. Bacteriophage-derived templates, such as M13 or other filamentous phage DNA, can be engineered to contain the gene of interest under an appropriate promoter, offering another method for producing template DNA for IVT. For small-scale synthesis or short mRNA transcripts, synthetic oligonucleotides that include the necessary promoter and coding region can be used directly in IVT reactions.

A fourth aspect relates to a use of the molecular complex according to the present invention for in vitro transcription, wherein the capping efficiency is at least 80%, 81%, 82%, 83%, 84%, 85%, 86%, 87%, 88%, 89%, 90%, 91%, 92%, 93%, 94%, 95%, 96%, 97%, 98%, 99%, or up to 100%.

In the present invention, the capping efficiency is measured according to the methodology described in Example 8.

A fifth aspect relates to a method of producing at least one m⁷G-capped mRNA molecule, the method comprising: (i) providing a reaction mixture comprising the Cap analog as defined in the first aspect or any embodiment thereof, the DNA template and/or the vector and/or the DNA molecule according to the present invention and at least one T7 RNA polymerase and/or the sequence encoding the same; (ii) allowing in vitro transcription from said DNA template and/or from said vector and obtaining at least one m7G-capped mRNA; (iv) optionally: purifying said at least one m7G-capped mRNA; and/or (v) optionally: formulating said at least one m7G-capped mRNA molecule yielding a pharmaceutical composition.

**Example 3** describes one preferable method of producing the at least one m7G-capped mRNA molecule.

One embodiment relates to the method of the fifth aspect further comprising a step of: (vii) introducing or contacting said at least one m7G-capped mRNA molecule obtained in step (iii) into or with at least one target cell in an in vitro cellular system for transfecting said at least one target cell and/or for in vitro studying the functionality of said at least one m7G-capped mRNA.

A sixth aspect relates to a pharmaceutical composition for use in a method of preventing and/or treating a disease, the pharmaceutical composition comprising the at least one m7G-capped mRNA molecule obtained or obtainable by the method according to the fifth aspect or the embodiment thereof, additionally comprising at least one pharmaceutically acceptable carrier or excipient.

In certain embodiments, the pharmaceutically acceptable carrier or excipient comprises at least one bulking agent and/or at least one binder and/or at least one disintegrant and/or at least one coating and/or at least one filler or diluent and/or at least one stabilizer or preservative and/or at least one solvent and/or at least one solubilizer.

One of the primary functions of a pharmaceutically acceptable carrier or excipient is to provide the necessary bulk and form to create various dosage forms, including suspensions, or injectable solutions, ensuring the integrity of the final product. Additionally, carriers or excipients play a pivotal role in enhancing the stability of the active pharmaceutical ingredient (API), shielding it from factors like light, heat, moisture, or chemical reactions that could lead to degradation.

A critical aspect of these carrier or excipients is their ability to adjust the pH of the formulation to levels conducive to the stability and efficacy of the API. Importantly, these substances must not interact adversely with the API or other components, ensuring the safety and integrity of the medicinal product. Pharmaceutically acceptable carriers and excipients adhere to stringent quality and safety standards, ensuring they do not introduce impurities, contaminants, or adverse effects into the pharmaceutical product. They are indispensable in drug development, allowing for the creation of formulations suitable for patient administration, ultimately delivering the desired therapeutic effect while upholding safety and stability standards.

One embodiment relates to the pharmaceutical composition for use in a method of preventing and/or treating a disease according to the sixth aspect, the pharmaceutical composition additionally comprising at least one lipid nanoparticle, wherein the at least one lipid nanoparticle is selected from the group of at least one liposome (LPs), at least one liposome-like nanoparticle (LLP), at least one solid lipid nanoparticle (SLN), at least one nanostructured lipid carrier (NLC), at least one lipid-polymer hybrid nanoparticle (LPN), at least one lipoprotein particle (LPT), at least one nanoemulsion, at least one cationic nanoemulsion (CNE) and at least one exosome.

Lipid nanoparticles (LNPs) are fundamental components in the field of mRNA delivery, playing a crucial role in the transportation and protection of mRNA molecules for a variety of biotechnological and therapeutic applications.

One of the primary functions of LNPs is to provide a protective encapsulation for mRNA. This encapsulation shields the mRNA from degradation by nucleases, ensuring the genetic information remains intact during transportation. Additionally, LNPs facilitate the efficient cellular uptake of mRNA by cells, typically through endocytosis, allowing the encapsulated mRNA to enter the cytoplasm where translation and protein synthesis occur.

LNPs contribute to the stability and bioavailability of mRNA therapeutics, safeguarding the mRNA cargo from degradation by the recipients' immune system and enhancing its pharmacokinetics. Thus, an essential attribute of LNPs is their minimal immunogenicity when properly designed, reducing the risk of undesirable immune responses against the delivery system itself. Furthermore, they can be engineered for tissue-specific targeting, enabling the targeting of specific cell types or organs for therapeutic purposes.

A seventh aspect relates to a kit comprising the Cap analog according to the present invention, optionally comprising the DNA template according to the present invention, and/or the vector and/or the DNA molecule according to the present invention, optionally wherein the kit comprises at least one further reagent and/or at least one aqueous solution, preferably a buffer.

Whenever the present disclosure relates to the percentage of identity of nucleic acid or amino acid sequences to each other these values define those values as obtained by using the EMBOSS Water Pairwise Sequence Alignments (nucleotide) programme (https://www.ebi.ac.uk/idispatcher/psa/emboss water ) nucleic acids or the EM-BOSS Water Pairwise Sequence Alignments (protein) programme (www.ebi.ac.uk/Tools/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see https://www.ebi.ac.uk/jdispatcher/psa and SMITH, T.F. 5 & WATERMAN, M.S. "Identification of common molecular subsequences" Journal of Molecular Biology, 1981 147 (1):195-197). When conducting an alignment, the default parameters defined by the EMBL-EBI are used.

Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a nucleotide sequence encoding a protein can be "codon optimized" if the respective sequence is to be used in another organism in comparison to the original organism a molecule originates from.

The present invention is further illustrated by the following, non-limiting examples.

### Examples

### Example 1 Synthesis of tetranucleotide cap analogs.

### Example 1A: General aspects

The tetranucleotide cap analogs were synthesized by modified previously reported synthesis of di- and trinucleotide cap analogues. Warminski et al. "Trinucleotide mRNA Cap Analogue N6-Benzylated at the Site of Posttranscriptional m6Am Mark Facilitates mRNA Purification and Confers Superior Translational Properties In Vitro and In Vivo" J. Am. Chem. Soc. vol. 146, pp. 8149-8163 (2024).) The multistep synthesis of tetranucleotide cap analogs sodium salts consisted of: solid-phase synthesis (SPS) of 5'-phosphorylated trinucleotide, its activation with imidazole and coupling with 7-methylguanosine 5'-diphosphate analogue followed by ion-exchange DEAD Sephadex chromatography, purification by RP HPLC (C18) and multiple freeze drying with addition of NaHCOS aqueous solution. The N6-benzyladenosine phosphoramidite and N6-methyladenosine phosphoramidite for solid-phase synthesis were prepared according to previously described protocols. See Warminski et al. "Quick Access to Nucleobase-Modified Phosphoramidites for the Synthesis of Oligoribonucleotides Containing PostTranscriptional Modifications and Epitranscriptomic Marks" J. Org. Chem. vol. 87, pp. 10333-10348 (2022).

General procedure for synthesizing 5'-monophosphorylated ribotrinucleotides (A*pG*pG or A*pA*pG): Synthesis was performed in a 50 mL glass solid-phase vessel equipped with a frit filter. First coupling: Primer Support 5G RNA (308/303 µmol/g, GE Healthcare) resin was placed in SPS vessel and it was sealed with Precision Seal^{®} rubber septa, and purged with argon. Then a 0.3 M solution of appropriate phosphoramidite (1.5 equiv, G, Gm, A, Am) in dry acetonitrile and BTT activator (2.25 equiv, 0.3 M 5-(benzylthio)-1H-tetrazole solution in acetonitrile) were added. The suspension was gently shaken for 1 hour at room temperature. The resin was washed with acetonitrile, 3% (w/v) trichloroacetic acid solution in dichloromethane (detritylation), 0.05 M iodine in pyridine/water (9:1) (oxidation), acetonitrile and dried under vacuum for 1 hour. Second and third coupling was performed in the same manner as first, using 1.5 equiv of A, Am, Bn6¬Am, m6A phosphoramidite or 2.5 equiv of bis(2-cyanoethyl)-N,N-diisopropyl phosphoramidite. After the last coupling, trinucleotides, still attached to the solid support, were washed with 20% (v/v) diethylamine in acetonitrile, acetonitrile and dried under vacuum. Trinucleotides were cleaved from solid support and deprotected by AMA solution (methylamine/ammonium hydroxide 1:1 (v/v)) at 55 °C for 1 h. Solution was evaporated. The TBDMS were removed using of TEA·3HF (1.55 equiv), TEA (1.55 equiv) in dry DMSO (0.1 M). Mixture was stirred at 60 °C for 1 h. The mixture was 10x diluted with MQ water. The product was isolated by ion-exchange chromatography on DEAE Sephadex (elution gradient: 0-1.2 M TEAB buffer) to afford trinucleotides as triethylammonium salts. The synthesis scales, chemical structure, yield, HPLC and LRMS data for particular trinucleotides are summarized in **Table 1** and **2.**

**Table 1. 5'-Phosphorylated trinucleotide analogs.**

| Trinucleot ide | Chemical structure | SPS scale [µmol] | Yield^{[} a] [µmo l] | HPLC Rₜ^{[b]} [min] | Mass spectrometry | | |
|---|---|---|---|---|---|---|---|
| | | | | | Calculate d for | Calcul ated m/z | Measur ed m/z^{[c]} |
| p^{bn6}AₘpG pG | 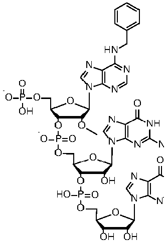 | 200 | 138 | 8.438 | C₃₈H₄₄N₁₅O₂₁P₃²⁻ | 569.9 | 569.7 |
| pAₘpGₘp G | 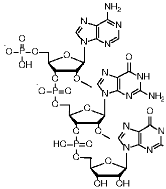 | 1200 | 826 | 5.979 | C₃₂H₄₀N₁₅ O₂₁P₃²⁻ | 531.6 | 532.4 |

**Table 2. Structure of synthesized tetranucleotide cap analogs.**

| |
|---|
| m⁷Gppp^{bn6}AₘpGpG: |
| 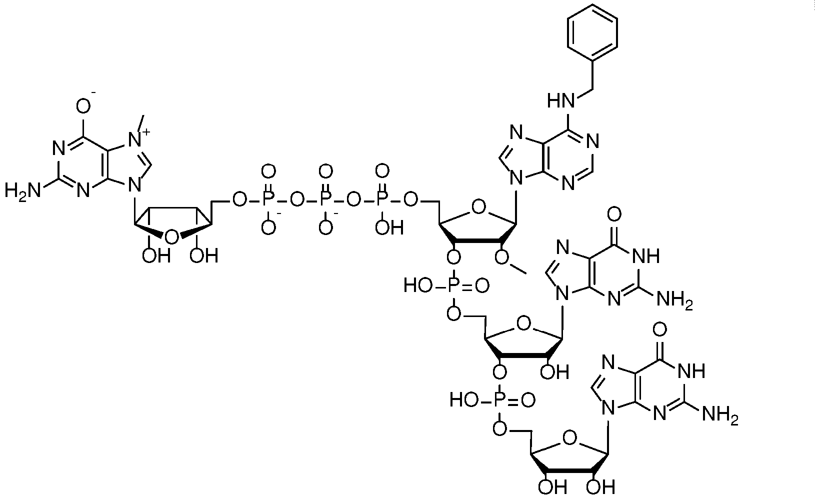 |
| m⁷GpppAₘpGₘpG: |
| 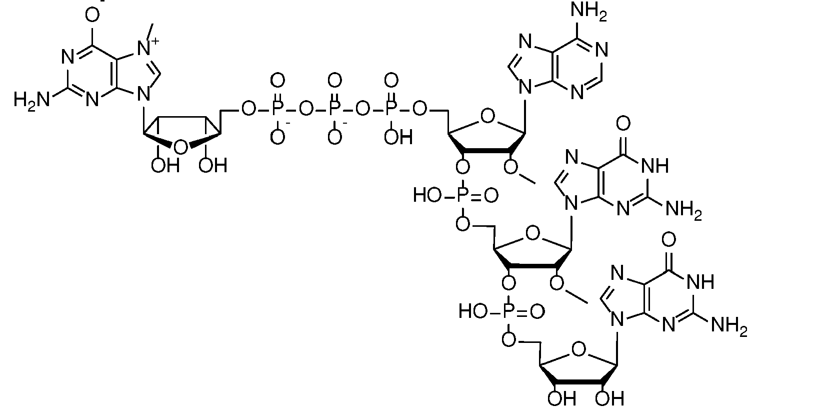 |
| m₂^{7,2'O}GpppAₘGₘG: |
| 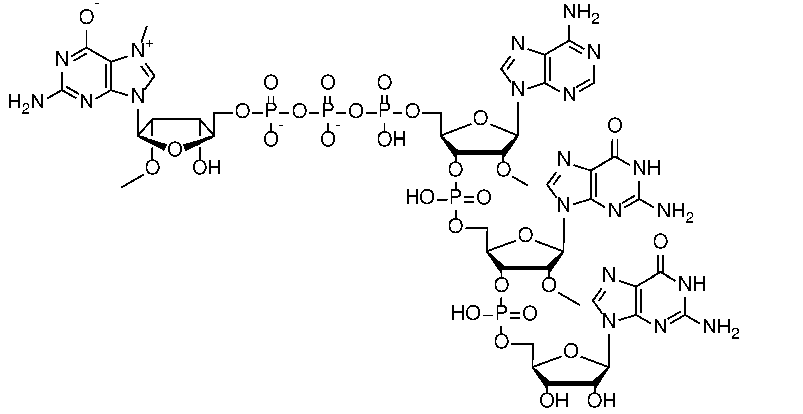 |
| m⁷Gppp^{bn6}AₘpGₘpG: |
| 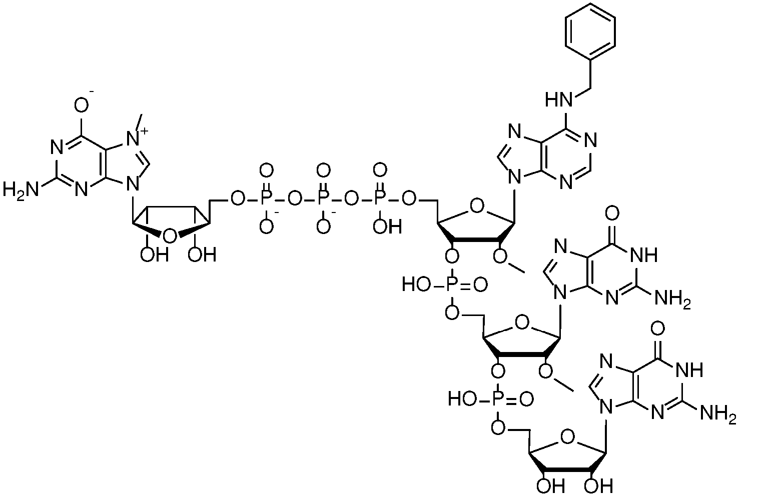 |
| m⁷GpppApGₘpG: |
| 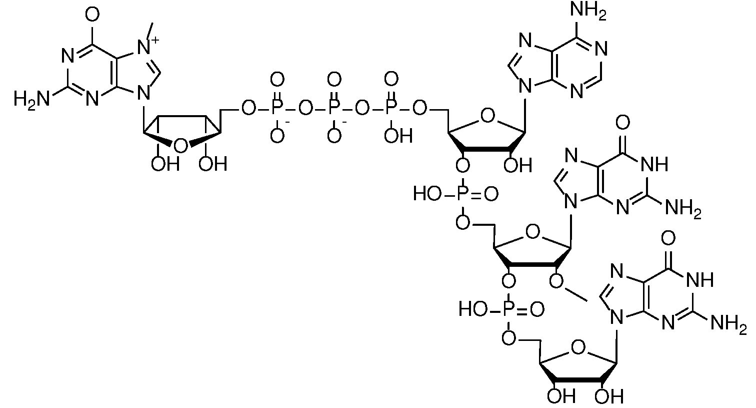 |
| m⁷Gppp^{m6}ApGₘpG: |
| 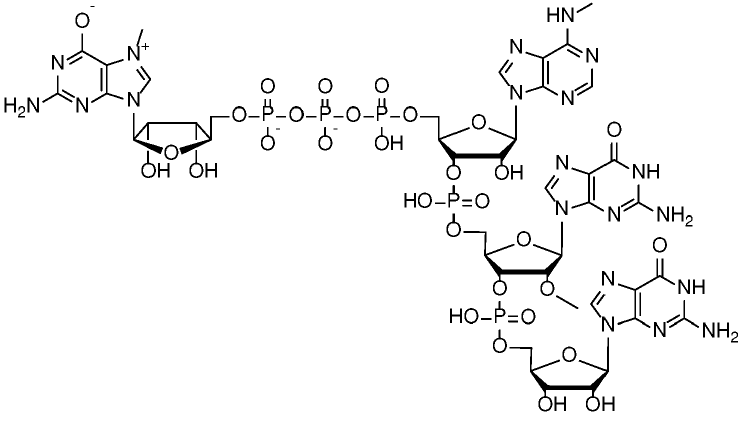 |
| m⁷GpppAₘpGpG: |
| 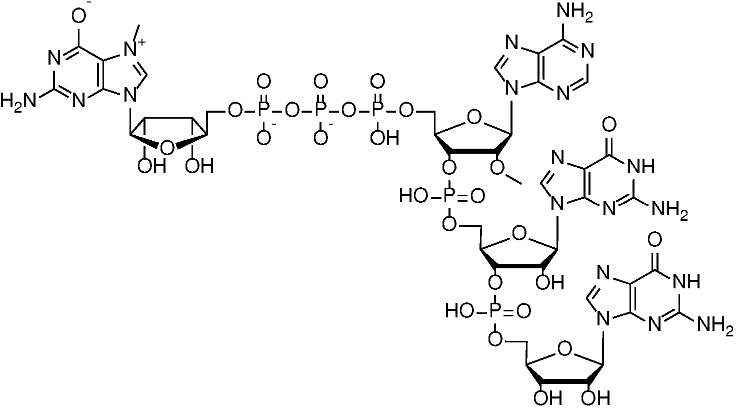 |
| m⁷GpppAₘpAₘpG: |
| 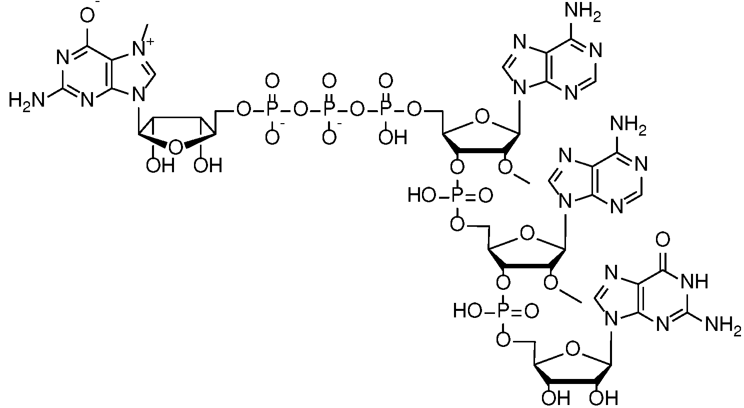 |
| m⁷Gppp^{bn6}AₘpApG: |
| 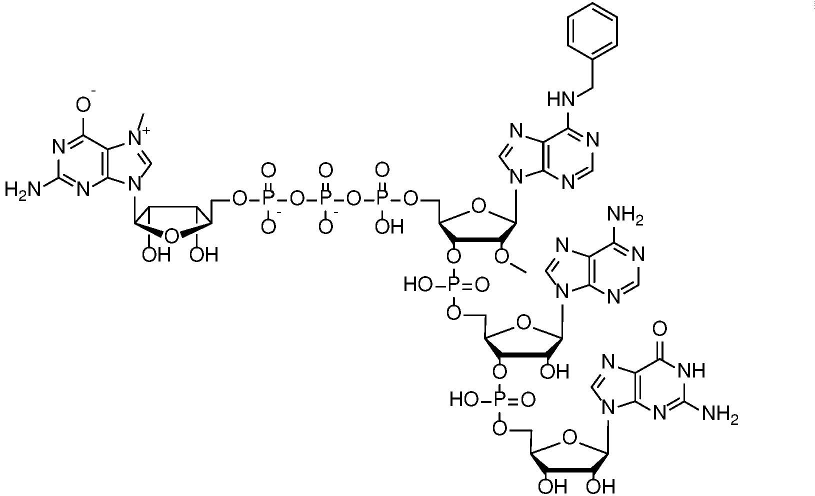 |
| m⁷GpppAₘpApG: |
| 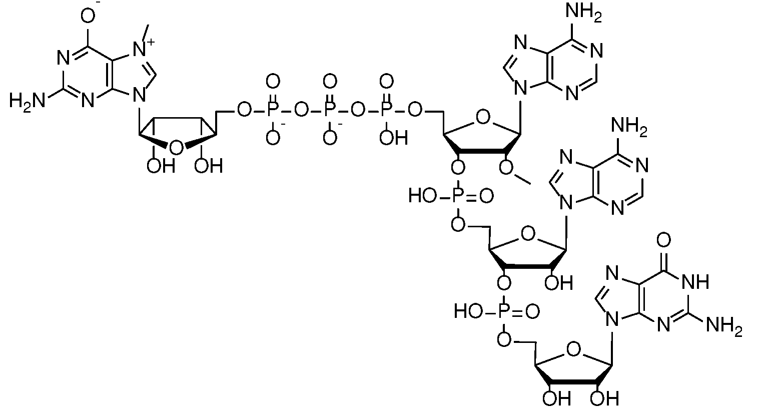 |
| m⁷Gppp^{bn6}AₘpAₘpG: |
| 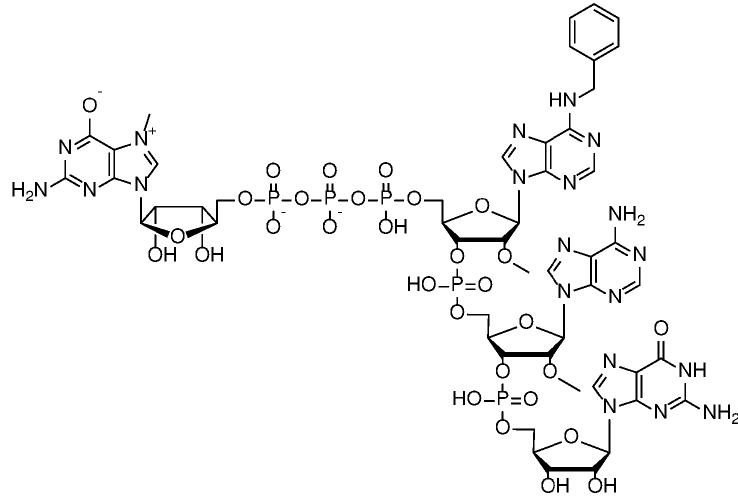 |

### Example 1B: General procedure for synthesis of tetranucleotide cap analogs:

Step A: Synthesis of phosphorimidazolide intermediate: To a 0.05 M solution of trinucleotide 5'phosphate triethylammonium salt in dry DMF, 2,2'-dithiodipiridine (6 equiv), imidazole (16 equiv) and triethylamine (6 equiv) were added. Mixture was stirred at room temperature for 15 minutes. Then triphenylphosphine (6 equiv) was added. The clear yellow solution was stirred at room temperature till full conversion of the trinucleotide (progress on RP-HPLC, 1-24 h). The product was precipitated by addition of cold solution of NaClO₄ (10 equiv) in acetone (10 times the volume of DMF used). The precipitate was centrifuged (4 °C), washed twice with cold acetonitrile and dried overnight under vacuum. The phosphorimidazolide intermediate without further purification was used in next step.

Step B: Coupling reaction of activated trinucleotide with m⁷GDP/TEA or m₂^{7,2'O}GDP/TEA: The phosphorimidazolide derivative of trinucleotide (1 equiv) was added to a DMF solution of triethylammonium salt of 7-methylguanosine 5'-diphosphate (m⁷GDP/TEA) or 2'O-modified 7-methylguanosine 5'-diphosphate (m₂^{7,2'O}GDP/TEA) (1.2 equiv) and ZnCl₂ (12 equiv). The reaction mixture was stirred for 1-4 hours, then it was quenched by adding a aqueous solution (10 times the volume of DMF used) of Na₂EDTA (50 mg/mL) and NaHCOs (25 mg/mL). The product was isolated by ion-exchange chromatography (DEAE Sephadex, gradient elution 0-1.2 M TEAB) and then purified by RP-HPLC (C18, linear gradient of acetonitrile in aqueous ammonium acetate buffer). After lyophilization tetranuclotide cap analogs were obtained as ammonium salts.

Step C: Exchange of tetranuclotide cap analogs ammonium salts to sodium salts: To an aqueous solution of ammonium salts of tetranuclotide caps (0.04 M) an aqueous solution of NaHCOs (1 M, 3.5 equiv) was added. Mixture was vortexed, centrifuged and freeze-dried twice to get sodium salts of tetranuclotide cap analogs as colourless powder.

m⁷Gppp^{bn6}AₘpGpG: Total yield: 27%. RP-HPLC: *Rₜ* = 7.959 min. ¹H NMR (500 MHz, D₂O, 70 °C) δ 9.07 (s, 1H), 8.48 (s, 1H), 8.24 (s, 1H), 8.07 (s, 1H), 8.05 (s, 1H), 7.44-7.28 (m, 5H), 6.02 (d, J = 6.2 Hz, 1H), 5.91 (d, J = 4.3 Hz, 1H), 5.85 (d, J = 5.4 Hz, 1H), 5.80 (d, J = 5.9 Hz, 1H), 4.94-4.89 (m, 1H), 4.88-4.84 (m, 3H), 4.82-4.77 (m, 1H), 4.70 (t, J = 5.4 Hz, 1H), 4.62 (t, J = 4.3 Hz, 1H), 4.50-4.40 (m, 6H), 4.25-4.13 (m, 7H), 4.03 (s, 3H), 3.34 (s, 3H). ³¹P NMR (203 MHz, D₂O, 70 °C): δ = 0.82 (s, 1P), 0.40 (s, 1P), -10.06 (d, J = 19.0 Hz, 1P), -10.18 (d, J = 18.4 Hz, 1P), -21.52 (dd, J = 19.0, 18.4 Hz, 1P). LRMS ESI(-): *m*/*z* 789.2 (*calcd for* C₄₉H₅₉N₂₀O₃₁P₅²⁻ [M-2H]²⁻ 789.1).

m⁷GpppAₘpGₘpG: Total yield: 23%. RP-HPLC: *Rₜ* = 5.469 min; ¹H NMR (500 MHz, D₂O) δ 9.42 (s, 1H), 8.83 (s, 1H), 8.56 (s, 1H), 8.37 (s, 1H), 8.31 (s, 1H), 6.31 (d, J = 5.5 Hz, 1H), 6.23 (d, J = 4.0 Hz, 1H), 6.16 (d, J = 5.4 Hz, 1H), 6.12 (d, J = 6.0 Hz, 1H), 5.20 (ddt, J = 8.0, 5.0, 3.2 Hz, 2H), 5.04 (t, J = 5.4 Hz, 1H), 4.93 (dd, J = 5.0, 4.0 Hz, 1H), 4.91 - 4.88 (m, 1H), 4.82 - 4.74 (m, 3H), 4.66 - 4.59 (m, 3H), 4.57 - 4.53 (m, 1H), 4.48 (ddt, J = 17.0, 12.1, 4.1 Hz, 5H), 4.35 (s, 3H), 3.69 (s, 3H), 3.66 (s, 3H). ³¹P NMR (203 MHz, D₂O) δ -0.49 (s, 1P), -0.67 (s, 1P), -10.70 - -11.50 (m, 2P), -22.38 - -22.59 (m, 1P). LRMS ESI(-): *m*/*z* 750.8 (*calcd for* C₄₃H₅₅N₂₀O₃₁P₅²⁻ [M-2H]²⁻ 751.1).

m₂^{7,2'O}GpppAₘpGₘpG: Total yield: 22% RP-HPLC: *Rₜ* = 5.527 min; 1H NMR (500 MHz, D₂O) δ 9.08 (s, 1H), 8.46 (s, 1H), 8.18 (s, 1H), 8.01 (s, 1H), 7.87 (s, 1H), 5.97 (d, J = 4.8 Hz, 1H), 5.91 (d, J = 3.0 Hz, 1H), 5.86 (d, J = 5.6 Hz, 1H), 5.73 (d, J = 6.0 Hz, 1H), 4.93-4.86 (m, 2H), 4.63 (t, J = 5.6 Hz, 1H), 4.55 (t, J = 5.5 Hz, 1H), 4.49 (dd, J = 5.2, 3.9 Hz, 1H), 4.45 (d, J = 3.7 Hz, 1H), 4.42 - 4.07 (m, 12H), 4.05 (s, 3H), 3.54 (s, 3H), 3.39 (d, J = 7.2 Hz, 6H), 2.04 (s, 1H), 2.00 (s, 1H). ³¹P NMR (203 MHz, D₂O) δ 0.09 (s, 1P), -0.08 (s, 1P), -10.52 (d, J = 18.6 Hz, 1P), -10.64 (d, J = 17.9 Hz, 1P), -21.91 (t, J = 18.1 Hz, 1P). LRMS ESI(-): *m*/*z* 757.8 (*calcd for* C₄₄H₅₇N₂₀O₃₁P₅²⁻ [M-2H]²⁻ 758.1).

m⁷Gppp^{bn6}AₘpGₘpG: Total yield: 37%. RP-HPLC: *Rₜ* = 7.737 min; ¹H NMR (500 MHz, D₂O) δ 9.36 (s, 1H), 8.75 (s, 1H), 8.33 (s, 1H), 8.30 (s, 1H), 7.68 - 7.52 (m, 6H), 6.31 (d, J = 5.9 Hz, 1H), 6.18 (d, J = 4.0 Hz, 1H), 6.13 (t, J = 6.2 Hz, 2H), 5.21 (dq, J = 8.2, 3.9 Hz, 2H), 5.11 (s, 2H), 5.02 (t, J = 5.4 Hz, 1H), 4.92 - 4.85 (m, 2H), 4.83 - 4.72 (m, 6H), 4.61 (qd, J = 4.6, 1.9 Hz, 3H), 4.56 - 4.43 (m, 5H), 4.31 (s, 3H), 3.69 (s, 3H), 3.66 (s, 3H), 2.28 (s, 1H). ³¹P NMR (203 MHz, D₂O) δ -0.49 (s, 1P), -0.64 (s, 1P), -11.12 (t, J = 18.7 Hz, 2P), -22.51 (t, J = 18.0 Hz, 1P). LRMS ESI(-): *m*/*z* 796.3 (*calcd for* C₅₀H₆₁N₂₀O₃₁P₅²⁻ [M-2H]²⁻ 796.2). m⁷GpppApGₘpG: Total yield: 36%. RP-HPLC: *Rₜ* = 4.903 min; ¹H NMR (500 MHz, D₂O, 60 °C) δ 8.69 (s, 1H), 8.45 (s, 1H), 8.27 (s, 1H), 8.21 (s, 1H), 6.23 (d, *J =* 6.0 Hz, 1H), 6.20 (d, *J* = 4.0 Hz, 1H), 6.13 (dd, *J =* 5.7, 2.5 Hz, 2H), 5.21 (s, 1H), 5.09 - 5.01 (m, 2H), 4.99 (d, *J* = 5.6 Hz, 1H), 4.90 (t, *J =* 4.5 Hz, 1H), 4.84 (t, *J =* 5.5 Hz, 1H), 4.76 (t, *J =* 4.8 Hz, 4H), 4.60 (s, 4H), 4.48 (s, 8H), 4.33 (s, 3H), 4.22 - 4.09 (m, 1H), 3.69 (s, 3H). ³¹P NMR (203 MHz, D₂O, 60 °C) δ -0.37 (s, 1P), -0.46 (s, 1P), -11.10 (dd, *J* = 18.2, 8.0 Hz, 2P), -22.55 (t, *J* = 18.6 Hz, 1P). LRMS ESI(-): *m*/*z* 744.5 (*calcd for* C₄₂H₅₃N₂₀O₃₁P₅²⁻ [M-2H]²⁻ 745.1).

m⁷Gppp^{m6}ApGₘpG: Total yield: 37%. RP-HPLC: *Rₜ* = 5.170 min; ¹H NMR (500 MHz, D₂O, 60 °C) δ 8.70 (s, 1H), 8.50 (s, 1H), 8.42 - 8.21 (m, 2H), 6.28 (dd, *J =* 15.2, 4.9 Hz, 2H), 6.20 (dd, *J =* 5.9, 2.6 Hz, 2H), 5.32 - 5.22 (m, 1H), 5.19 - 5.03 (m, 5H), 5.00 - 4.89 (m, 10H), 4.72 - 4.47 (m, 5H), 4.39 (s, 3H), 3.76 (s, 3H), 3.49 (s, 3H). ³¹P NMR (203 MHz, D₂O, 60 °C) δ 0.53 (d, *J =* 15.7 Hz), -9.71 - -10.53 (m), -21.63 (t, *J =* 18.6 Hz). LRMS ESI(-): *m*/*z* 751.3 *(calcd for* C₄₃H₅₅N₂₀O₃₁P₅²⁻ [M-2H]²⁻ 751.5).

m⁷GpppAₘpGpG: Total yield: 23%. RP-HPLC: *R_{f}* = 4.765 min; ¹H NMR (500 MHz, D₂O, 60 °C) δ 9.48 (s, 1H), 8.88 (s, 1H), 8.63 (s, 1H), 8.41 (s, 1H), 8.34 (s, 1H), 6.38 (d, J = 5.6 Hz, 1H), 6.30 (d, J = 3.9 Hz, 1H), 6.22 (d, J = 5.3 Hz, 1H), 6.14 (d, J = 5.8 Hz, 1H), 5.28 (dt, J = 8.1, 4.1 Hz, 1H), 5.22 (t, J = 5.6 Hz, 1H), 5.16 (dt, J = 8.4, 4.3 Hz, 1H), 5.08 (t, J = 5.4 Hz, 1H), 5.00 (t, J = 4.5 Hz, 1H), 4.85 (p, J = 5.0 Hz, 3H), 4.74 - 4.66 (m, 2H), 4.66 - 4.48 (m, 6H), 4.42 (s, 3H), 3.74 (s, 3H). ³¹P NMR (203 MHz, D₂O, 60 °C) δ 0.74 (s, 1P), 0.30 (s, 1P), -10.17 (dd, J = 26.8, 18.2 Hz, 2P), -21.55 (t, J = 18.1 Hz, 1P). LRMS ESI(-): *m*/*z* 743.9 (*calcd for* C₄₂H₅₃N₂₀O₃₁P₅²⁻ [M-2H]²- 744.2).

m⁷GpppAₘpAₘpG: Total yield: 42%. RP-HPLC: *Rₜ =* 5.842 min; ¹H NMR (500 MHz, D₂O) δ 8.41 (s, 1H), 8.26 (s, 1H), 8.22 (s, 1H), 7.95 (s, 1H), 7.89 (s, 1H), 6.02 (d, J = 3.7 Hz, 1H), 5.93 (d, J = 4.7 Hz, 1H), 5.74 (dd, J = 6.8, 4.5 Hz, 2H), 4.91 - 4.82 (m, 2H), 4.64 (t, J = 5.2 Hz, 1H), 4.50 (dt, J = 8.7, 4.7 Hz, 2H), 4.43 (ddt, J = 15.9, 10.1, 5.2 Hz, 4H), 4.34 (ddt, J = 13.6, 6.0, 3.8 Hz, 6H), 4.27 - 4.17 (m, 7H), 3.98 (s, 3H), 3.55 (s, 3H), 3.52 (s, 3H). ³¹P NMR (203 MHz, D₂O) δ -0.05 (s, 1P), -0.30 (s, 1P), -10.49 (d, J = 18.6 Hz, 1P), -10.70 (d, J = 17.7 Hz, 1P), -21.83 (t, J = 18.0 Hz, 1P). LRMS ESI(-): *m*/*z* 742.8 (*calcd for* C₄₂H₅₃N₂₀O₃₁P₅²⁻ [M-2H]²⁻ 744.2).

m⁷Gppp^{bn6}AₘpApG: Total yield: 36%. RP-HPLC: *Rₜ* = 7.788 min; ¹H NMR (500 MHz, D₂O) δ 9.05 (s, 1H), 8.42 (s, 1H), 8.30 (s, 1H), 8.24 (s, 1H), 7.93 (d, J = 7.6 Hz, 2H), 7.24 - 7.15 (m, 2H), 7.18 (s, 3H), 5.92 (dd, J = 13.8, 4.6 Hz, 2H), 5.83 (d, J = 3.7 Hz, 1H), 5.77 (d, J = 5.0 Hz, 1H), 4.88 (dt, J = 8.3, 4.2 Hz, 1H), 4.68 - 4.59 (m, 2H), 4.58 - 4.42 (m, 7H), 4.38-4.12 (m, 7H), 4.02 - 3.98 (m, 3H), 3.51 (s, 3H). ³¹P NMR (203 MHz, D₂O) δ 0.12 (s, 1P),-0.18 (s, 1P), -10.51 (t, J = 19.3 Hz, 2P), -21.83 (t, J = 18.0 Hz, 1P). LRMS ESI(-): *m*/*z* 781.4 *(calcd for* C₄₉H₅₉N₂₀O₃₀P₅²⁻ [M-2H]²⁻ 781.1).

m⁷GpppAₘpApG: Total yield: 17%. RP-HPLC: *Rₜ* = 4.865 min; ¹H NMR (500 MHz, D₂O) δ 9.05 (s, 1H), 8.48 (s, 1H), 8.30 (s, 1H), 8.24 (s, 1H), 7.99 (s, 1H), 7.89 (s, 1H), 6.04 - 5.87 (m, 2H), 5.71-5.67 (m 2H), 4.94 - 4.84 (m, 1H), 4.67 - 4.60 (m, 1H), 4.58 - 4.10 (m, 13H), 3.96 (s, 3H), 3.57 (s, 3H). ³¹P NMR (203 MHz, D₂O) δ 0.15 (s, 1P), -0.47 (s, 1P), -10.45 (d, J = 18.1 Hz, 1P), -10.87 (d, J = 17.3 Hz, 1P), -21.62 (t, J = 17.6 Hz, 2P). LRMS ESI(-): *m*/*z* 735.8 (*calcd for* C₄₂H₅₃N₂₀O₃₀P₅²⁻ [M-2H]²⁻ 736.1).

m⁷Gppp^{bn6}AₘpAₘpG: Total yield: 16%. RP-HPLC: *Rₜ* = 8.979 min; ¹H NMR (500 MHz, D₂O) δ 9.37 (s, 1H), 8.79 (s, 1H), 8.77 (s, 1H), 8.64 (s, 1H), 8.45 (s, 1H), 8.36 (s, 1H), 7.64 - 7.45 (m, 5H), 6.37 (d, J = 5.2 Hz, 1H), 6.28 (d, J = 6.5 Hz, 1H), 6.19 (d, J = 4.0 Hz, 1H), 6.13 (d, J = 5.4 Hz, 1H), 5.25 (dt, J = 8.4, 4.5 Hz, 1H), 5.19 (ddd, J = 7.5, 4.7, 2.6 Hz, 1H), 5.08 (s, 2H), 5.01 (t, J = 5.3 Hz, 1H), 4.89 (t, J = 4.5 Hz, 1H), 4.85 - 4.74 (m, 5H), 4.60 (dq, J = 5.2, 3.2 Hz, 3H), 4.56 - 4.42 (m, 8H), 4.31 (s, 3H), 3.74 (s, 3H), 3.64 (s, 3H). ³¹P NMR (203 MHz, D₂O) δ -0.09 (s, 1P), -0.21 (s, 1P), -10.54 (dd, J = 28.5, 18.5 Hz, 2P), -21.76 (t, J = 16,1 Hz, 1P). LRMS ESI(-): *m*/*z* 788.5 (*calcd for* C₅₀H₆₁N₂₀O₃₀P₅²⁻ [M-2H]²⁻ 788.2).

### Example 2: General procedure for templates preparation.

Wild type (WT) or optimized luciferase gene from firefly (Lampyridae) (FFLuc) subcloned into pJET1.2 plasmid vector was linearized using type IIS restriction enzyme, e.g. Eam1104I or Aarl (ThermoFisher Scientific) generating 5' overhanges. Completeness of the linearization was verified on the 1×TBE 1% agarose gel and the linearized plasmid was purified using a commercial DNA purification kit (Macherey-Nagel).

PCR templates were prepared using pJET1.2-FFLuc WT or pJET1.2-FFLuc optimized circular plasmids as a templates, Platinum^{™} SuperFi^{™} II DNA Polymerase (Invitrogen^{™}), forward primer covering T7 promoter, TSS and several additional nucleotides downstream 5' end, and reverse primer containing polyT sequence and several additional nucleotides downstream 3'UTR. Next, the PCR mix was purified using GeneJET PCR Purification Kit (ThermoFisher Scientific) and template purity was verified on the 1×TBE 1.2% agarose gel.

### Example 3: General procedure for in vitro transcription of mRNAs using tetranucleotide primers m⁷GpppA*pG*pG and m⁷GpppA*pA*pG. see also FIG 5, FIG 7-11

The dsDNA template encoding FFLuc was prepared by plasmid linearization (see also **Example 2)** at the site following polyA tail-coding region. Three different templates were used for these experiments: 1) template T1 (Φ6.5-AGG), which contained <t>6.5 promoter followed by AGG deoxyribonucleotides at positions +1, +2 and +3 of the coding strand (in this case non-coding strand contained deoxyribonucleotides TTCC corresponding to the positions -1, +1, +2 and +3); 2) template T2 (Φ2.5-AGG), which contained <t>2.5 promoter followed by AGG nucleotides at positions +1, +2 and +3 of the coding strand (in this case non-coding strand contained deoxyribonucleotides ATCC corresponding to the positions-1, +1, +2 and +3); 3) template T3 (Φ6.5-GGG), which contained <t>6.5 promoter followed by GGG nucleotides at positions +1, +2 and +3 of the coding strand (in this case non-coding strand contained deoxyribonucleotides TCCC corresponding to the positions -1, +1, +2 and +3). In vitro transcription reactions for 10, 5 and 2 mM of tetranucleotide primer m⁷GpppAₘpGₘpG, m⁷GpppAₘpAₘpG, m⁷GpppAₘpGpG, m⁷GpppAₘpApG, m⁷Gppp^{Bn6}AₘpGₘpG, m⁷Gppp^{Bn6}AₘpAₘpG, m⁷Gppp^{Bn6}AₘpGpG or m⁷Gppp^{Bn6}AₘpApG and for 10, 5 and 2 mM Bn⁶-containing trinucleotide primer m⁷Gppp^{Bn6}AₘpG were prepared using 40 mM Bis-Tris buffer pH 6.5, containing 2 mM spermidine, and 5 mM each of ATP, UTP, CTP, 4 mM GTP, 25 mM MgCl₂, 10 mM DTT, 40 ng/µL linearized plasmid as the DNA template, 1 U/µL RNase inhibitor (RiboLock, ThermoFisher Scientific), 0.002 U/µL inorganic pyrophosphatase (ThermoFisher Scientific) and 75 U/µL T7 RNA polymerase (Roche). In vitro transcription reactions for 10, 5 and 2 mM of trinucleotide cap 1 primer m⁷GpppAₘpG were prepared using 40 mM Tris buffer pH 8.0, containing 2 mM spermidine, and 5 mM each of ATP, UTP, CTP, 4 mM GTP, 10 mM MgCl₂, 10 mM DTT, 40 ng/µL linearized plasmid as the DNA template, 1 U/µL RNase inhibitor (RiboLock, ThermoFisher Scientific), 0.002 U/µL inorganic pyrophosphatase (ThermoFisher Scientific) and 75 U/µL T7 RNA polymerase (Roche). For reactions providing uncapped mRNAs, cap analog was omitted. All mixed components were incubated for 2h at 37°C, and subsequently treated with 0.025 U/µL of DNase I (ThermoFisher Scientific) for 30 min at 37°C. The enzymes in the IVT mixtures were inactivated by addition of one volume of 50 mM EDTA, and full-length mRNAs were purified by affinity chromatography (described in **Example 6)** with oligo(dT)₂₅ resin (POROS^{™} Oligo (dT)₂₅ Affinity Resin, ThermoFisher Scientific).

### Example 4: General procedure for in vitro transcription of mRNAs with m⁷GpppA*pU*pG tetranucleotide primers; see FIG 12, FIG 14

The dsDNA template encoding firefly luciferase was prepared by PCR (see also **Example 2).** Two different templates were used for this experiment: 1) template T4 (Φ6.5-ATG), which contained <t>6.5 promoter followed by ATG deoxyribonucleotides at positions +1, +2 and +3 of the coding strand (in this case non-coding strand contained deoxyribonucleotides TTAC corresponding to the positions -1, +1, +2 and +3); 2) template T5 (Φ6.5-TGG), which contained <t>6.5 promoter followed by TGG nucleotides at positions +1, +2 and +3 of the coding strand (in this case non-coding strand contained deoxyribonucleotides TACC corresponding to the positions -1, +1, +2 and +3). In vitro transcription reactions for 8 mM of tetranucleotide primer m⁷GpppAₘpUₘpG, m⁷GpppAₘpUpG, m⁷Gppp^{Bn6}AₘpUₘpG, m⁷Gppp^{Bn6}AₘpUpG, m⁷GpppAₘpAₘpU, trinucleotide cap 1 primer m⁷GpppAₘpU and Bn⁶-modified trinucleotide primer m⁷Gppp^{Bn6}AₘpU were prepared using 40 mM Bis-Tris buffer pH 6.5, containing 2 mM spermidine, and 5 mM each of ATP, UTP, CTP and GTP, 30 mM MgCl₂, 10 mM DTT, 20 ng/µL PCR-generated DNA template, 1 U/µL RNase inhibitor (RiboLock, ThermoFisher Scientific), 0.002 U/µL inorganic pyrophosphatase (ThermoFisher Scientific) and 75 U/µL T7 RNA polymerase (Roche). For reactions providing uncapped mRNAs, cap analog was omitted. All mixed components were incubated for 2h at 37°C, and subsequently treated with 0.025 U/µL of DNase I (ThermoFisher Scientific) for 30 min at 37°C. The enzymes in the IVT mixtures were inactivated by addition of one volume of 50 mM EDTA, and full-length mRNAs were purified by affinity chromatography (described in **Example 6)** with oligo(dT)₂₅ resin (POROS^{™} Oligo (dT)₂₅ Affinity Resin, ThermoFisher Scientific).

### Example 5: General procedure for in vitro transcription of mRNAs with m⁷GpppA*pG*pA tetranucleotide primers; see FIG 15, FIG 16

The dsDNA template encoding firefly luciferase was prepared by PCR (see also **Example 2).** Two different templates were used for this experiment: 1) template T6 (Φ6.5-AGA), which contained <t>6.5 promoter followed by AGA deoxyribonucleotides at positions +1, +2 and +3 of the coding strand (in this case non-coding strand contained deoxyribonucleotides TTCT corresponding to the positions -1, +1, +2 and +3); 2) template T7 (Φ6.5-GAG), which contained <t>6.5 promoter followed by GAG nucleotides at positions +1, +2 and +3 of the coding strand (in this case non-coding strand contained deoxyribonucleotides TCTC corresponding to the positions -1, +1, +2 and +3). In vitro transcription reactions for 10 mM of tetranucleotide primer m⁷GpppAₘpGₘpA, m⁷GpppAₘpGpA, m⁷Gppp^{Bn6}AₘpGₘpA or m⁷Gppp^{Bn6}AₘpGpA, and for Bn⁶-containing trinucleotide primer m⁷Gppp^{Bn6}AₘpG were prepared using 40 mM Bis-Tris buffer pH 6.5, containing 2 mM spermidine, and 5 mM each of ATP, UTP, CTP, 4 mM GTP, 25 mM MgCl₂, 10 mM DTT, 20 ng/µL PCR-generated DNA template, 1 U/µL RNase inhibitor (RiboLock, ThermoFisher Scientific), 0.002 U/µL inorganic pyrophosphatase (ThermoFisher Scientific) and 75 U/µL T7 RNA polymerase (Roche). In vitro transcription reactions for trinucleotide cap 1 primer m⁷GpppAₘpG were prepared using 40 mM Tris buffer pH 8.0, containing 2 mM spermidine, and 5 mM each of ATP, UTP, CTP, 4 mM GTP, 10 mM MgCl₂, 10 mM DTT, 20 ng/µL PCR-generated DNA template, 1 U/µL RNase inhibitor (RiboLock, ThermoFisher Scientific), 0.002 U/µL inorganic pyrophosphatase (ThermoFisher Scientific) and 75 U/µL T7 RNA polymerase (Roche). For reactions providing uncapped mRNAs, cap analog was omitted. All mixed components were incubated for 2h at 37°C, and subsequently treated with 0.025 U/µL of DNase I (ThermoFisher Scientific) for 30 min at 37°C. The enzymes in the IVT mixtures were inactivated by addition of one volume of 50 mM EDTA, and full-length mRNAs were purified by affinity chromatography (described in **Example 6)** with oligo(dT)₂₅ resin (POROS^{™} Oligo (dT)₂₅ Affinity Resin, ThermoFisher Scientific).

### Example 6: mRNA purification using oligo(dT)₂₅ resin.

Each crude IVT mix inactivated with 50 mM EDTA was further diluted with high salt buffer (1200 mM KCI, 10 mM Tris-HCI pH 7.5, 1 mM EDTA) and loaded on the accordingly equilibrated oligo(dT)₂₅ resin. After binding step (10 min, RT), resin was washed with high and low salt buffer (200 mM KCl, 10 mM Tris-HCI pH 7.5, 1 mM EDTA), respectively. mRNA was eluted with RNase-free water heated to 65°C, and prior IVT efficiency estimation and ribozyme treatment it was concentrated by ultrafiltration (Amicon Ultra-0.5 50K, Millipore).

### Example 7: Determination of in vitro transcription efficiency.

IVT efficiencies were estimated spectrophotometrically after mRNA purification on the oligo(dT)₂₅ resin and concentration by ultrafiltration using Amicon Ultra-0.5 50K (Millipore) filtration devices. After recovering concentrated mRNA, sample volume and the absorbance at 260 nm corresponding to mRNA concentration was measured using NanoDrop One^{C} (ThermoFisher Scientific) spectrophotometer. IVT yield was calculated by multiplying the volume of the concentrated sample and measured mRNA concentration expressed as ng/µL. IVT efficiency was calculated as mRNA yield obtained from 1 µL of starting IVT volume.

### Example 8: Determination of capping efficiency; see FIG 5, FIG 7-13, FIG 12, FIG 15

Ribozyme complementary to the 5'UTR sequence was designed and used for cleavage of mRNA close to the 5' end. 10 µL of analyzed sample containing >10 µg (>100 nM) of mRNA was mixed with 1.5 µL of hybridization buffer containing 100 mM Tris-HCI pH 7.5 and 50 mM NaCl and 2 µL of 10 µM ribozyme. The mixture was incubated at 95°C for 2 min, and subsequently at RT for 10-15 min. Cleavage reaction with the ribozyme hybridized to mRNA was initiated by addition of 1.5 µL of 100 mM MgCl₂, conducted at 37°C for 1h, and quenched by addition of 2 µL 100 mM EDTA.

To analyze cleaved RNA fragments 3 µL of quenched reaction was mixed with equal volume of loading dye (8 M urea, 50% formamide, 20 mM EDTA, 0.03% bromophenol blue, 0.03% xylene cyanol), heat denatured at 95°C for 3 min and loaded onto 15% polyacrylamide gel with 7 M urea and 1× TBE. The gel after electrophoresis was incubated at RT for 15 min with 50 mL of staining reagent (SYBR^{®} Gold, Invitrogen, diluted 1 :10000), and visualized using GelDoc Go Imaging System (Bio-Rad). Migration of short 5' RNA fragments being the result of ribozyme cleavage differs depending on the cap structure present on the 5' end of mRNA. Additionally, bands intensities correlate with the amount of particular RNA species. The intensity of the bands corresponding with capped and uncapped RNA (ppp-RNA) were quantified densitometrically using ImageQuantTL software (GE Healthcare). Percentage of capped mRNA in each sample was determined as the ratio of capped RNA and the summarized intensities for capped RNA and ppp-RNA.

### Example 9: Analysis and Results of IVT yield and capping efficiency analysis.

We have employed three dsDNA templates (T1-T3) encoding Firefly luciferase, carrying either <t>6.5 or <t>2.5 promoter and AGG or GGG sequence at +1, +2, and +3 positions and tested different initiating primers, respectively **(****FIG 1****).** We first tested these templates against two different cap 2 primers (m⁷GpppAₘpGₘpG or m⁷GpppAₘpAₘpG), whose synthesis is shown in **FIG 2****.** These primers were designed to be fully complementary to positions +1, +2 and +3 or -1, +1 and +2, respectively, of the DNA template T1 (Φ6.5-AGG), and are fully or partially complementary to the other templates **(****FIG 4A to D****).** Additionally, an IVT reaction with a tetranucleotide cap 1 primer designed by analogy to what Ishikawa has proposed for trinucleotides was performed (Ishikawa et al., 2009, *supra*)*.* The tetranucleotide primer (m⁷GpppAₘGG) was designed as complementary (starting from the position -1) to the Ishikawa's type template i.e. containing φ6.5 promoter followed by GGG (T3).

The mRNAs were synthesized by in vitro transcription in the presence of 5 mM each of ATP, UTP, CTP, 4 mM GTP, 10 mM cap analogs, and 25 mM MgCl₂ as described in Example ## The mRNAs were purified by affinity chromatography (described in **Example 6).** Purified mRNA was cleaved at the 5' ends by a ribozyme, and analyzed by electrophoresis followed by densitometry to determine the capping efficiencies **(Example 8),** as described previously.(Vlatkovic et al., Pharmaceutics, 2022, 14(2):328; doi: 10.3390/pharmaceutics14020328)] IVT yields were determined spectrophotometrically after mRNA purification by measuring the volume of concentrated eluate and the absorbance at 260 nm corresponding to mRNA concentration. **(Example 7).** Uncapped mRNA, which was used as a reference, was synthesized in the same way, except that no cap analog was added to the IVT mix.

**FIG 5** shows representative results of such an experiment. Uncapped mRNAs produced from all templates (T1-T3) produce a major RNA product of 23 nt in length after the ribozyme-mediated cleavage. This confirms that the transcription start sites (+1 positions) for templates T1-T3 are as indicated in **FIG 4A** and **B****.** Some additional bands corresponding to longer and shorter RNAs are also visible for these samples, which is a common phenomenon for in vitro transcribed RNA obtained with T7 polymerase(Pomerantz et al., Mol Cell, 2006, 20;24(2):245-55)In the presence of cap analogs, additional bands corresponding to longer RNAs are visible. These bands represent capped RNAs of various lengths (24-26 nt). The lengths of the major observed capped RNA bands correspond well with the anticipated hybridization modes in the transcription initiation complexes **(****FIG 6****).** The densitometric quantification of band intensities enabled us to determine the overall capping efficiencies, which are also shown in **FIG 5****.** The results show a clear advantage of using m⁷GpppAₘpAₘpG as a transcription primer over m⁷GpppAₘpGₘpG for each of the studied templates.

For template T1 (Φ6.5-AGG), m⁷GpppAₘpGₘpG afforded 90% capping efficiency, whereas for m⁷GpppAₘpAₘpG, uncapped RNA was virtually not observed (capping efficiency -100%). Moreover, we observed that using m⁷GpppAₘpAₘpG resulted in mRNA of much higher homogeneity of the 5' end. A virtually single band was observed for the capped RNA species obtained with m⁷GpppAₘpAₘpG. In contrast, for m⁷GpppAₘpGₘpG two distinctive RNA species were observed, which may result either from initiation of transcription at alternative sites or insertion of additional nucleotides by T7 polymerase (Pleiss et al., RNA, 1998;4(10):1313-7).

For templates T2 (Φ2.5-AGG) and T3 (Φ6.5-GGG), we generally observed lower capping efficiencies than for template T1 (Φ6.5-AGG), but in both cases m⁷GpppAₘpAₘpG afforded 20-30% higher capping efficiency than m⁷GpppAₘpGₘpG and higher homogeneity of the capped RNA species, despite comparable or even lower degree of complementarity to the template **(****FIG 6****).** This is in contrast to what has been previously claimed by WO2017053297A1 that the complementarity between the template and the primer at positions +1 +2 (+3) and N¹, N² (and N³), respectively, is the main prerequisite for achieving high capping efficiency and 5' end homogeneity. Also, it was found that the transcription with m⁷GpppAₘpAₘpG from Φ6.5-AGG template T1 **(****FIG 5****;** lane 6) significantly improved capping efficiency and 5' end homogeneity without compromising the IVT yield compared to the results obtained for cap 1 analogs and following the approach previously proposed by Ishikawa **(****FIG.5****,** lanes 10 and 11).

We next expanded this experiment to study various derivatives of m⁷GpppA*pG*pG or m⁷GpppA*pA*pG (wherein A* and G* denote adenosine or guanosine moieties, unmodified or modified at the positions 2'-O and Bn⁶ to form various cap 1 and cap 2 analogs) against the same set of templates. The representative results for templates T1, T2, and T3 (see **Table 4)** and eight cap analogs (four derivatives of m⁷GpppA*pG*pG type and four corresponding of m⁷GpppA*pA*pG type) are shown in **FIG 7****,** **8** and **9****,** respectively, and the mean results of replicate experiments are shown in **Tables 5-7.** These experiments confirmed our initial finding that the combination of the template T1 (Φ6.5-AGG) and m⁷GpppA*pA*pG type initiator gives the best capping efficiency results without compromising the in vitro transcription yield. **FIG 7** shows that combination of any of the studied m⁷GpppA*pA*pG derivatives with template T1 (Φ6.5-AGG) affords capping efficiency close to 100%. This is the case even for tetranucleotide analogs carrying bulky benzyl group at the N6-position of adenosine, which affects the capping efficiency for the corresponding trinucleotide (maximum capping efficiency is 92% at pH 6.5, and decreases with increasing pH (Warminski et al., Journal of the American Chemical Society2024 146(12), 8149-8163). In contrast, the mRNAs obtained in the presence of m⁷GpppA*pG*pG derivatives as transcription initiators under the same conditions are characterized by higher 5' end heterogeneity and capping efficiencies that do not exceed 90%, and decrease notably for analogs carrying bulkier modifications (81% and 84% for ^{m7}Gppp^{Bn6}AₘpGpG and ^{m7}Gppp^{Bn6}AₘpGₘpG, respectively). Moreover, the presence of the Bn⁶ modification within m⁷GpppA*pG*pG analogs significantly decreased the in vitro transcription yield. Overall, we discovered that the combination of m⁷GpppA*pA*pG, but not m⁷GpppA*pG*pG, with template T1 (Φ6.5-AGG) enables preparation of mRNAs comprising cap 1 and cap 2 structures, either unmodified or carrying additional chemical modifications, with exceptionally high capping efficiency and 5' end homogeneity and with very good in vitro transcription yield.

**Table 3. Tetrameric and trimeric capped oligonucleotides used as the transcription initiation primers in the in vitro transcription experiments**

| Figure/lane ID# | Cap analog | Alternative abbreviation |
|---|---|---|
| FIG 7-11/1 | ^{m7}Gppp^{Bn6}AₘpGpG | Cap 1 ^{Bn6}AGG |
| FIG 7*-*11/2, FIG 15/1, 5 | ^{m7}Gppp^{Bn6}AₘpApG | Cap 1 ^{Bn6}AAG |
| FIG 5/2, FIG 7-11/3 | m⁷GpppAₘpGₘpG | Cap 2 AGG |
| FIG 7-11/4 | ^{m7}Gppp^{Bn6}AₘpGₘpG | Cap 2 ^{Bn6}AGG |
| FIG 5/3, FIG 7-11/5, FIG 15/2, 6 | ^{m7}GpppAₘpAₘpG | Cap 2 AAG |
| FIG 7-11/6, FIG 15/3, 7 | ^{m7}GpppAₘpApG | Cap 1 AAG |
| FIG 7-11/7, FIG 15/4, 8 | ^{m7}Gppp^{Bn6}AₘpAₘpG | Cap 2 ^{Bn6}AAG |
| FIG 5/10 | ^{m7}GpppAₘpGpG | Cap 1 AGG |
| FIG 7-11/8 | | |
| FIG 5/11 | m⁷GpppAₘpG | Cap 1 AG |
| FIG 7-11/9, FIG 15/11, 13, FIG 16/11, 13 | | |
| FIG 7-11/10, FIG 15/12, 14, FIG 16/12, 14 | ^{m7}Gppp^{Bn6}AₘpG | Cap 1 ^{Bn6}AG |
| FIG 14/2, FIG 12/1, 4 | ^{m7}GpppAₘpUpG | Cap 1 AUG |
| FIG 14/4, FIG 12/2, 5 | ^{m7}GpppAₘpUₘpG | Cap 2 AUG |
| FIG 14/3 | ^{m7}Gppp^{Bn6}AₘpUpG | Cap 1 ^{Bn6}AUG |
| FIG 14/1 | ^{m7}Gppp^{Bn6}AₘpUₘpG | Cap 2 ^{Bn6}AUG |
| FIG 14/5, FIG 12/3, 6 | m⁷GpppAₘpAₘpU | Cap 2 AAU |
| FIG 14/6 | ^{m7}GpppAₘpU | Cap 1 AU |
| FIG 14/7 | ^{m7}Gppp^{Bn6}AₘpU | Cap 1 ^{Bn6}AU |
| FIG 16/1, 5 | ^{m7}GpppAₘpGpA | Cap 1 AGA |
| FIG 16/2, 6 | ^{m7}Gppp^{Bn6}AₘpGpA | Cap 1 ^{Bn6}AGA |
| FIG 16/3, 7 | ^{m7}GpppAₘpGₘpA | Cap 2 AGA |
| FIG 16/4, 8 | ^{m7}Gppp^{Bn6}AₘpGₘpA | Cap 2 ^{Bn6}AGA |

**Table 4. Promoters and trinucleotide initiating sequences of the DNA templates used in the experiments**

| Template ID | Data shown in Figure | Promoter-¹TSS | ²Template nucleotides at positions: -1\|+1\|+2\|+3 | CDS |
|---|---|---|---|---|
| T1 | FIG 5, FIG 7, FIG 10, FIG 11, FIG 15 | Φ6.5-AGG | 3'...T\|T\|C\|C...5' | FFLuc |
| T2 | FIG 8 | Φ2.5-AGG | 3'...A\|T\|C\|C...5' | FFLuc |
| T3 | FIG 9 | Φ6.5-GGG | 3'...T\|C\|C\|C...5' | FFLuc |
| T4 | FIG 12, FIG 14 | Φ6.5-ATG | 3'...T\|T\|A\|C...5' | FFLuc |
| T5 | FIG 12, FIG 14 | Φ6.5-TGG | 3'...T\|A\|C\|C...5' | FFLuc |
| T6 | FIG 16 | Φ6.5-AGA | 3'...T\|T\|C\|T...5' | FFLuc |
| T7 | FIG 16 | Φ6.5-GAG | 3'...T\|C\|T\|C...5' | FFLuc |
| T8 | FIG 15 | Φ6.5-AAG | 3'...T\|T\|T\|C...5' | FFLuc |

| | | | | |
|---|---|---|---|---|
| ¹TSS - transcription start site (sequence of the coding strand of the dsDNA template). First transcribed nucleotide which corresponds to the position +1 of the template strand is underlined. ² nucleotides of the non-coding strand following core promoter region of the dsDNA template; positions +1+2+3 represent the TIS starting with the TSS at +1. | | | | |

**Table 5. Summarized results of replicate experiments: in vitro transcription (IVT) and capping efficiency (±SD) for tetrameric and trimeric capped oligonucleotide primers (10 mM) used in the experiments with optimized IVT conditions (pH 6.5) and template T1 (<t>6.5-AGG, see also FIG 4)**

| Lane ID in **FIG 7** | Cap analog (alternative abbreviation) | IVT efficiency [µg/µL] (±SD) | Capping efficiency [%] (±SD) |
|---|---|---|---|
| 1 | ^{m7}Gppp^{Bn6}AₘpGpG (Cap 1 Bn6AGG) | 0.85 (0.01) | 82.00 (1.41) |
| 2 | ^{m7}Gppp^{Bn6}AₘpApG (Cap 1 ^{Bn6}AAG) | 4.36 (0.16) | 98.00 (0.00) |
| 3 | m⁷GpppAₘpGₘpG (Cap 2 AGG) | 4.25 (0.17) | 88.50 (0.71) |
| 4 | ^{m7}Gppp^{Bn6}AₘpGₘpG (Cap 2 ^{Bn6}AGG) | 1.50 (0.05) | 84.50 (0.71) |
| 5 | m⁷GpppAₘpAₘpG (Cap 2 AAG) | 4.04 (0.33) | 100.00 (0.00) |
| 6 | ^{m7}GpppAₘpApG (Cap 1 AAG) | 4.10 (0.18) | 100.00 (0.00) |
| 7 | ^{m7}Gppp^{Bn6}AₘpAₘpG (Cap 2 ^{Bn6}AAG) | 4.02 (0.35) | 100.00 (0.00) |
| 8 | ^{m7}GpppAₘpGpG (Cap 1 AGG) | 3.98 (0.42) | 89.50 (0.71) |
| 9 | ^{m7}GpppAₘpG (Cap 1 AG) | 4.20 (0.37) | 100.00 (0.00) |
| 10 | ^{m7}Gppp^{Bn6}AₘpG (Cap 1 ^{Bn6}AG) | 3.59 (0.23) | 93.00 (1.41) |

**Table 6. Summarized results of replicate experiments: in vitro transcription (IVT) and capping efficiency (±SD) for tetrameric and trimeric capped oligonucleotide primers (10 mM) used in the experiments with optimized IVT conditions (pH 6.5) and template T2 (<t>2.5-AGG, see also FIG 4)**

| Lane ID in **FIG 8** | Cap analog (alternative abbreviation) | IVT efficiency [µg/µL] (±SD) | Capping efficiency [%] (±SD) |
|---|---|---|---|
| 1 | ^{m7}Gppp^{Bn6}AₘpGpG (Cap 1 ^{Bn6}AGG) | 3.20 (0.67) | 72.00 (5.66) |
| 2 | ^{m7}Gppp^{Bn6}AₘpApG (Cap 1 ^{Bn6}AAG) | 4.30 (0.12) | 96.00 (0.00) |
| 3 | ^{m7}GpppAₘpGₘpG (Cap 2 AGG) | 4.32 (0.01) | 84.00 (1.41) |
| 4 | ^{m7}Gppp^{Bn6}AₘpGₘpG (Cap 2 ^{Bn6}AGG) | 3.81 (0.01) | 80.00 (0.00) |
| 5 | ^{m7}GpppAₘpAₘpG (Cap 2 AAG) | 3.96 (0.11) | 100.00 (0.00) |
| 6 | ^{m7}GpppAₘpApG (Cap 1 AAG) | 3.96 (0.21) | 100.00 (0.00) |
| 7 | ^{rn7}Gppp^{Bn6}AₘpAₘpG (Cap 2 ^{Bn6}AAG) | 4.20 (0.01) | 100.00 (0.00) |
| 8 | ^{m7}GpppAₘpGpG (Cap 1 AGG) | 4.08 (0.07) | 83.00 (0.00) |
| 9 | ^{m7}GpppAₘpG (Cap 1 AG) | 3.55 (0.95) | 100.00 (0.00) |
| 10 | ^{m7}Gppp^{Bn6}AₘpG (Cap 1 ^{Bn6}AG) | 3.96 (0.40) | 90.50 (3.54) |

**Table 7. Summarized results of replicate experiments: in vitro transcription (IVT) and capping efficiency (±SD) for tetrameric and trimeric capped oligonucleotide primers (10 mM) used in the experiments with optimized IVT conditions (pH 6.5) and template T3 (<t>6.5-GGG, see also FIG 4)**

| Lane ID in **FIG 9** | Cap analog (alternative abbreviation) | IVT efficiency [µg/µL] (±SD) | Capping efficiency [%] (±SD) |
|---|---|---|---|
| 1 | ^{m7}Gppp^{Bn6}AₘpGpG (Cap 1 ^{Bn6}AGG) | 2.97 (0.22) | 66.50 (2.12) |
| 2 | ^{m7}Gppp^{Bn6}AₘpApG (Cap 1 ^{Bn6}AAG) | 4.02 (0.06) | 77.00 (2.83) |
| 3 | ^{m7}GpppAₘpGₘpG (Cap 2 AGG) | 4.27 (0.36) | 54.00 (1.41) |
| 4 | ^{m7}Gppp^{Bn6}AₘpGₘpG (Cap 2 ^{Bn6}AGG) | 4.03 (0.23) | 72.50 (2.12) |
| 5 | ^{m7}GpppAₘpAₘpG (Cap 2 AAG) | 4.33 (0.40) | 71.00 (0.00) |
| 6 | ^{m7}GpppAₘpApG (Cap 1 AAG) | 4.27 (0.31) | 80.00 (0.00) |
| 7 | ^{m7}Gppp^{Bn6}AₘpAₘpG (Cap 2 ^{Bn6}AAG) | 3.90 (0.08) | 65.00 (1.41) |
| 8 | ^{m7}GpppAₘpGpG (Cap 1 AGG) | 4.21 (0.04) | 66.50 (3.54) |
| 9 | ^{m7}GpppAₘpG (Cap 1 AG) | 3.97 (0.42) | 94.00 (0.00) |
| 10 | ^{m7}Gppp^{Bn6}AₘpG (Cap 1 ^{Bn6}AG) | 4.28 (0.25) | 90.50 (0.71) |

We additionally tested the same set of initiating nucleotides with template T2 (Φ2.5-AGG) under analogous conditions **(****FIG 8****).** The experiments revealed that transcriptions with primers derived from m⁷GpppA*pA*pG structure (m⁷GpppAₘpAₘpG, m⁷GpppAₘpApG, m⁷Gppp^{Bn6}AₘpAₘpG and m⁷Gppp^{Bn6}AₘpApG) result in mRNAs characterized by higher capping efficiencies than transcriptions with m⁷GpppA*pG*pG structure, despite the fact that only the latter is fully complementary to the template (as shown in **FIG 6****).** Also, the transcriptions performed in the presence of m⁷GpppA*pG*pG, especially Bn⁶-modified and cap 2 variants, resulted in significantly lower overall IVT yields and higher 5' end heterogeneities. This suggested that the alignment of the m⁷GpppA¹*pA²*pG tetranucleotide primer with the DNA template wherein the position A¹* of the primer is aligned with position -1 of the DNA template (even if not complementary to the template) is favorable over the alignment of m⁷GpppA*pG^{1*}pG²tetranucleotide wherein the position A* of the primer is aligned with the position +1 of the template (even if complementary to the template). The first case produces mRNA of higher capping efficiency and higher 5' end homogeneity and with higher overall yield, suggesting that the complexes formed between the T7 RNA polymerase, m⁷GpppA¹*pA²*pG-type cap analog, and the DNA template are more effective for transcription initiation.

Finally, we tested the same set of primers with template T3 (Φ6.5-GGG) **(****FIG 9****),** which provides incomplete complementarity for m⁷GpppA*pA*pG-type primers (2 out of 3 complementary base pairs) and differs in their alignment against the template **(****FIG 6****).** In this case we again observed that the m⁷GpppA*pA*pG-type primers provided significantly higher capping efficiencies than m⁷GpppA*pG*pG-type primers. Notably, the m⁷GpppA*pG*pG-type primers used with template T3 (Φ6.5-GGG) had an analogous alignment as m⁷GpppA*pA*pG-type with template T1 (Φ6.5-AGG), but yielded mRNAs characterized by significantly lower capping efficiencies and higher heterogeneities. This indicates that not only alignment position of the primer against the template, but also the sequence of the primer/template are a relevant factor for obtaining the most effective transcription initiation complexes. More importantly, it is ultimately the synthesis and the provision of a suitable synthetically modified tetranucleotide cap analogs and the targeted combination of these highly artificial cap analogs with a suitable cognate DNA template that will guarantee a high capping efficiency together with a high translational activity.

It was further demonstrated that the combination of m⁷GpppA*pA*pG-type primers and template T1 (Φ6.5-AGG) enables the preparation of high quality in vitro transcribed mRNA under more-restrictive conditions, particularly at decreased cap analog concentration. It is currently estimated that the cost of capping reagents is appr. 40% of the cost of production of in vitro transcribed mRNA. Therefore decreasing the concentration of cap analog in the IVT mix without compromising the capping efficiency, RNA integrity or IVT yield is highly desirable. As such we performed IVT reactions with template T1 (Φ6.5-AGG) in the presence of cap analogs at concentrations 5 mM or 2 mM. The representative results are shown in **FIG 10** (for 5 mM cap analog concentration) and **FIG 11** (for 2 mM cap analog concentration), respectively, and the mean results of replicate experiments are shown in **Table 8** and **9.** The experiments revealed excellent capping efficiencies for all studies tetranucleotide m⁷GpppA*pA*pG-type primers (cap 1 and cap 2 derivatives), despite cap analog concentration being decreased to 5 mM (capping efficiency 96%-100%) or even 2 mM (capping efficiency 90%-94%). Notably, for ^{m7}GpppA*pG*pG-type primers, capping efficiency is decreased to 76-86% (5 mM) and 60-76% (2 mM). Taking into account that the synthesis of tetranucleotide primers is not significantly more labor-intensive and reagent-consuming compared to trinucleotide capping reagents, the transcription initiation complexes comprising tetranucleotide cap 1 analogs according to this invention are also a viable alternative to complexes comprising trinucleotide cap 1 analogs.

**Table 8. Summarized results of replicate experiments: in vitro transcription (IVT) and capping efficiency (±SD) for tetrameric and trimeric capped oligonucleotide primers (5 mM) used in the experiments with optimized IVT conditions (pH 6.5) and template T1 (<t>6.5-AGG, see also FIG 4A)**

| Lane ID in FIG 10 | Cap analog (alternative abbreviation) | IVT efficiency [µg/µL] (±SD) | Capping efficiency [%] (±SD) |
|---|---|---|---|
| 1 | ^{m7}Gppp^{Bn6}AₘpGpG (Cap 1 ^{Bn6}AGG) | 3,20 (0,67) | 72,00 (5,66) |
| 2 | ^{m7}Gppp^{Bn6}AₘpApG (Cap 1 ^{Bn6}AAG) | 4,30 (0,12) | 96,00 (0,00) |
| 3 | ^{m7}GpppAₘpGₘpG (Cap 2 AGG) | 4,32 (0,01) | 85,00 (1,41) |
| 4 | ^{m7}Gppp^{Bn6}AₘpGₘpG (Cap 2 ^{Bn6}AGG) | 3,81 (0,01) | 80,00 (0,00) |
| 5 | ^{m7}GpppAₘpAₘpG (Cap 2 AAG) | 3,96 (0,11) | 100,00 (0,00) |
| 6 | ^{m7}GpppAₘpApG (Cap 1 AAG) | 3,96 (0,21) | 100,00 (0,00) |
| 7 | ^{m7}Gppp^{Bn6}AₘpAₘpG (Cap 2 ^{Bn6}AAG) | 4,20 (0,01) | 100,00 (0,00) |
| 8 | ^{m7}GpppAₘpGpG (Cap 1 AGG) | 4,08 (0,07) | 83,00 (0,00) |
| 9 | ^{m7}GpppAₘpG (Cap 1 AG) | 4,22 | 100,00 |
| 10 | ^{m7}Gppp^{Bn6}AₘpG (Cap 1 ^{Bn6}AG) | 4,24 | 88,00 |

**Table 9. Summarized results of the replicate experiments: in vitro transcription (IVT) and capping efficiency (±SD) for tetrameric and trimeric capped oligonucleotide primers (2 mM) used in the experiments with optimized IVT conditions (pH 6.5) and template T1 (<t>6.5-AGG, see also FIG 4A)**

| Lane ID in FIG 11 | Cap analog (alternative abbreviation) | IVT efficiency [µg/µL] (±SD) | Capping efficiency [%] (±SD) |
|---|---|---|---|
| 1 | ^{m7}Gppp^{Bn6}AₘpGpG (Cap 1 ^{Bn6}AGG) | 4,25 (0,02) | 61,50 (2,12) |
| 2 | ^{m7}Gppp^{Bn6}AₘpApG (Cap 1 ^{Bn6}AAG) | 4,21 (0,03) | 90,50 (0,71) |
| 3 | ^{m7}GpppAₘpGₘpG (Cap 2 AGG) | 4,15 (0,01) | 77,00 (1,41) |
| 4 | ^{m7}Gppp^{Bn6}AₘpGₘpG (Cap 2 ^{Bn6}AGG) | 4,26 (0,13) | 65,50 (2,12) |
| 5 | ^{m7}GpppAₘpAₘpG (Cap 2 AAG) | 3,86 (0,14) | 89,00 (1,41) |
| 6 | ^{m7}GpppAₘpApG (Cap 1 AAG) | 3,94 (0,01) | 93,00 (1,41) |
| 7 | ^{m7}Gppp^{Bn6}AₘpAₘpG (Cap 2 ^{Bn6}AAG) | 4,08 (0,01) | 88,50 (2,12) |
| 8 | ^{m7}GpppAₘpGpG (Cap 1 AGG) | 3,96 (0,02) | 71,50 (0,71) |
| 9 | ^{m7}GpppAₘpG (Cap 1 AG) | 4,07 (0,13) | 97,50 (0,71) |
| 10 | ^{m7}Gppp^{Bn6}AₘpG (Cap 1 ^{Bn6}AG) | 4,03 (0,08) | 72,00 (1,41) |

### Example 10: Analysis and Results of IVT yield and capping efficiency analysis for AUG cap analogs.

To explore alternative TSS combined with appropriate tetranucleotide cap primers we have employed two PCR-generated templates encoding FFLuc, carrying <t>6.5 promoter followed by ATG (template T4) or TGG (template T5) sequence **(****FIG 4C** and **D****).** Particularly, ATG trinucleotide initiating sequence is used in the constructs encoding self-amplifying mRNAs (saRNAs), being a promising improvement of the first generation of the mRNA-based vaccines. This solution provides comparable therapeutic effect obtained for decreased vaccination dose, which reduces manufacturing costs and potential harmful effects related to application of exogenous RNA [https://www.mdpi.com/2076-393X/12/3/318] It is expected that a similar therapeutic effect could be obtained for even lower dosages of saRNA capped with cap analogs carrying modifications increasing translational activity (such as Bn⁶), therefore, prolonging persistence of saRNA in cellular environment. As we discovered, co-transcriptional incorporation of such cap analogs by T7 RNA polymerase occurs only under the specific combination of transcription initiating primer and the DNA template containing particular TSS.

We first tested cap analogs m⁷GpppAₘpUₘpG and m⁷GpppAₘpUpG as the tetranucleotide primers designed to be fully complementary to templates T4 (Φ6.5-ATG) and and T5 (Φ6.5-TGG), but differing in respective alignments. Additionally, m⁷GpppAₘpAₘpU tetranucleotide primer was used with both templates (T4 and T5) to reflect full or partial complementarity previously observed for combination of m⁷GpppA*pA*pG-type primers with template T1 (<t>6.5-AGG) or template T3 (Φ6.5-GGG), respectively **(****FIG 6A** to D, **7** and **9).** The mRNAs were synthesized by in vitro transcription in the presence of 5 mM each of ATP, UTP, CTP and GTP, 8 mM cap analogs and 30 mM MgCl₂ as described in Example ##. The mRNAs were purified by affinity chromatography (described in Example 6). Purified mRNAs were cleaved at the 5' ends by a ribozyme, and analyzed by electrophoresis followed by densitometry to determine the capping efficiencies **(Example 8),** as described previously.[https://pubmed.ncbi.nlm.nih.gov/35214060/] IVT efficiencies were determined spectrophotometrically after mRNA purification by measuring the volume of concentrated eluate and the absorbance at 260 nm corresponding to mRNA concentration **(Example 7).** Uncapped mRNA, which was used as a reference, was synthesized in the same way, except that no cap analog was added to the IVT mix.

**FIG 12** depicts the obtained results. A first intriguing observation that we made was that uncapped RNAs obtained from these templates differed in length indicating most likely for different transcription initiation start sites. The result of ribozyme-mediated cleavage of uncapped RNA indicated that major initiation site for T5 was one nucleotide shorter than in case of the template T4 (Φ6.5-ATG) **(****FIG 12B****,** lane 7 vs. lane 8). In other words, the core promoter region was extended. Without wishing to be bound by theory, this confirms that the TSS (always numbered as +1 herein) for a given pair of primer::DNA template is dependent on the template and cannot be assumed to be in the same place for every template likely in view of steric aspects associated with the artificial tri- and tetranucleotide cap primers. For template T4, we observed the expected 23 nt-long uncapped product, analogously as in the case of IVT reactions performed for templates T1-T3. In the case of template T5 the product was 1 nt shorter indicating that the major transcription start site (+1) position has shifted to the guanine as indicated in **FIG 4****.** Such phenomenon is observed for the templates containing pyrimidine deoxyribonucleotide at position +1 of the coding strand, following core promoter region (Pomerantz et al., Mol Cell, 2006, 20;24(2):245-55).

However, bands on the denaturing gel for the transcripts obtained using template T5 (Φ6.5-TGG) and capped with tetranucleotides m⁷GpppA*pU*pG indicated formation of the initiation complex aligned analogously as shown in **FIG 13** with mRNA's 5' end being one nucleotide longer than in case of m⁷GpppA*pU*pG-capped transcripts obtained using template T4 (Φ6.5-ATG) **(****FIG 12B****,** lane 1 vs. lane 4 and lane 2 vs. lane 5). This result suggests that in the presence of tetranucleotide primer m⁷GpppA¹*pU²*pG³ T7 RNA polymerase recovers the expected register of transcription bubble and allows nucleotides U²* and G³ of the primer being fully hybridized with the template T5 (Φ6.5-TGG) of the non-coding strand.

Analyzing the results obtained in the presence of cap analogs we found that full complementarity between nucleotides N¹, N², and N³ of the tetranucleotide primer m⁷GpppAₘpUₘpG or m⁷GpppAₘpUpG with positions +1, +2 and +3 of the non-coding strand of template T4 (Φ6.5-ATG) guarantees high capping efficiency (100-95%). However, to the IVT yields for reaction were relatively low (especially for m⁷GpppAₘpUpG). The IVT efficiency was higher for m⁷GpppAₘpAₘpU analog, which is fully aligned with positions -1, +1 and +2 of the template T4 (Φ6.5-ATG), which also provided an excellent capping efficiency of 97%. The same set of tetranucleotide primers was efficiently (84-86%) incorporated into 5' ends of mRNAs encoded by the template T5 (Φ6.5-TGG) and allowed obtaining high transcription yield, even for previously ineffective m⁷GpppAₘUpG cap 1 analog.

We further expanded this experiment to investigate incorporation efficiency of heavily modified tetranucleotide analogs m⁷GpppA¹*pU²*pG³ (wherein A* and U* denote adenosine or uridine moieties, unmodified or modified at the positions 2'-O and Bn⁶ to form various cap 1 and cap 2 analogs) using the same set of DNA templates. These experiments confirmed that it is possible to co-transcriptionally introduce bulky substitutions (such as benzyl group at the N6-position of the adenosine A¹*) into 5' end of mRNA initiating with AUG sequence. However, to obtain the transcripts with heavily modified 5' ends, it is essential to design tetranucleotide cap primer:DNA template complex providing alignment of Bn6-modified adenosine A¹* with the position -1 (or -2 for T5) of the non-coding template strand **(****FIG 14****).** This observation combined with the previous findings described herein suggests that for efficient initiation of transcription with cap primers carrying bulky substitution, they should be located within T7 RNA polymerase binding pocket corresponding to -1 position of the non-coding DNA strand. Otherwise, when heavily modified cap analog is inappropriately aligned with the template, the transcriptional activity of the enzyme can be almost completely abolished **(****FIG 14B** vs. **FIG 14A****).**

Overall, we discovered that the combination of m⁷GpppA*pU*pG and m⁷GpppAₘpAₘpU with the template T5 (Φ6.5-TGG) enables preparation of mRNAs comprising cap 1 and cap 2 structures, either unmodified or carrying additional chemical modifications, with very good IVT yield, excellent 5' end homogeneity and with satisfactory capping efficiency, possibly improvable by additional optimization of the in vitro transcription reaction (e.g. by increasing cap analog concentration in the IVT mix).

### Example 11: Analysis and Results of IVT yield and capping efficiency analysis for heavily modified tetranucleotide cap analogs.

We discovered that for introduction of heavily modified cap structures into 5' end of RNA it is crucial to use appropriately designed tetranucleotide cap primer:DNA template complex, i.e. with Bn6-modified nucleotide N¹ being aligned with the position -1 (-2 for T5) of the non-coding template strand. To confirm these findings, we performed two additional experiments.

For this purpose, in the first experiment **(****FIG 15****)** we used previously validated combination of tetranucleotide primers m⁷GpppA*pA*pG (wherein A* and G* denote adenosine or guanosine moieties, unmodified or modified at the positions 2'-O and Bn⁶ to form various cap 1 and cap 2 analogs) with the template T1 (Φ6.5-AGG) (see **Table 4).** Additionally, the same set of tetranucleotide cap primers was applied for co-transcriptional capping using a newly designed template T8 (Φ6.5-AAG), which contained Φ6.5 promoter followed by AAG nucleotides at positions +1, +2 and +3 of the coding strand (in this case non-coding strand contained deoxyribonucleotides TTTC corresponding to the positions -1, +1, +2 and +3) (see **Table 4).** In the second experiment **(****FIG 16****)** we used the combination of two newly designed templates, i.e. T6 (Φ6.5-AGA) and T7 (Φ6.5-GAG), and variously compatible m⁷GpppA*pG*pA tetranucleotide cap analogs. Based on the discoveries described herein, we expect to observe full complementarity of m⁷GpppA*pG*pA cap primers with the positions +1, +2 and +3 of the template T6 (Φ6.5-AGA), and with the positions -1, +1 and +2 of the template T7 (Φ6.5-GAG), respectively.

In both experiments the mRNAs were synthesized as described in the **Example 11.** In vitro transcription was performed in the presence of 5 mM each of ATP, UTP, CTP, 4 mM GTP, 10 mM cap analogs, and 25 mM MgCl₂. The mRNAs were purified by affinity chromatography (described in **Example 6).** Purified mRNA was cleaved at the 5' ends by a ribozyme, and analyzed by electrophoresis followed by densitometry to determine the capping efficiencies **(Example 8),** as described previously. [https://pubmed.ncbi.nlm.nih.gov/35214060/] IVT efficiencies were determined spectrophotometrically after mRNA purification by measuring the volume of concentrated eluate and the absorbance at 260 nm corresponding to mRNA concentration **(Example 7).** Uncapped mRNA, which was used as a reference, was synthesized in the same way, except that no cap analog was added to the IVT mix.

As previously, m⁷GpppA*pA*pG tetranucleotide primers efficiently initiate transcription when nucleotides N¹, N² and N³ are aligned with positions -1, +1 and +2 of the template T1 (Φ6.5-AGG) **(****FIG 15****,** lanes 5-8, and **FIG 6A** to **D****).** Extraordinary capping efficiency and high IVT yield was obtained regardless bulky substituent present on the nucleotide N¹. On the other hand, for the template T8 (Φ6.5-AAG), which for m⁷GpppA*pA*pG-type analogs provides full complementarity of nucleotides N¹, N² and N³ with positions +1, +2 and +3 of the non-coding strand, efficiently capped RNA product was obtained only for tetranucleotides without Bn⁶ modification, i.e. m⁷GpppAₘpApG and m⁷GpppAₘpAₘpG (FIG **15****,** lane 2 and 3). Analogously, a satisfactory capping efficiency was obtained for all variants of m⁷GpppA*pG*pA tetranucleotides combined with template T7 (Φ6.5-GAG), providing alignment of nucleotides N¹, N² and N³ with positions -1, +1 and +2 of non-coding strand **(****FIG 16B****,** lanes 5-8 and **FIG 17****),** but transcription initiation with T6 (Φ6.5-AGA) template was efficient only for m⁷GpppA*pG*pA tetranucleotides lacking Bn⁶ modification **(****FIG 16B****,** lanes 1-4). Additionally, the 5' end of T7 (Φ6.5-GAG)-templated RNAs was more homogenous than for transcripts obtained using template T6 (Φ6.5-AGA), which enables hybridization of nucleotides N¹, N² and N³ with position +1, +2 and +3 of the template **(****FIG 16B****,** lanes 5-8 vs. lanes 1-4, respectively).

Overall, it was confirmed for various tetranucleotide cap primer:DNA template complexes **(Example 9, Example 10, Example 11),** that for efficient co-transcriptional capping, especially using heavily modified analogs (e.g. containing ^{Bn6}Aₘ modification, cap 2, and their combinations), it is essential to provide alignment of cap ribonucleotides N¹ and N² (N³) with position -1 and +1 (and +2) of the template strand (or -2, -1 and +1 for T5). Such hybridization pattern guarantees good capping efficiency, high IVT yield and increases homogeneity of the mRNA's 5' end.

## Claims

1. A molecular complex suitable for in vitro transcription with a T7 RNA polymerase comprising
(i) a Cap analog according to the general formula (I) 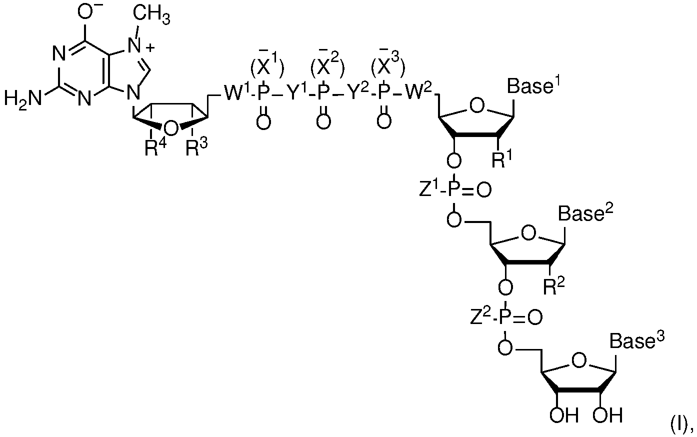
wherein Base¹, Base² and Base³ are independently a natural, unnatural or modified base;
R¹, R², R³ and R⁴ are independently OH, O-Me, H, F, Cl, O-alkyl, O-aryl, O-arylalkyl, O-acyl;
wherein at least one from R¹ and R², or both, is/are not OH; and X¹, X², X³ are independently O, S, BH₃, Se;
Y¹ and Y² are independently O, CH₂, CHCl, CHF, CF₂, CCl₂, NH;
W¹ and W² are independently O, S;
Z¹, Z² are independently O-, S-, CH₃, BH₃⁻;
(ii) a DNA template comprising a T7 polymerase promoter and a trinucleotide initiating sequence;
optionally: wherein nucleobase Base¹ hybridizes to the -1 position and/or preferably wherein nucleobase Base² hybridizes to the +1 position and Base³ hybridizes to the +2 position of the trinucleotide initiating sequence; or optionally: provided the DNA template comprises a core promoter of SEQ ID NO: 3 or the promoter comprises a a sequence of SEQ ID NOs: 12 or 13, nucleobase Base¹ hybridizes to the -2 position; nucleobase Base² hybridizes to the -1 position and Base³ hybridizes to the +1 position of the trinucleotide initiating sequence.

2. The molecular complex according to claim 1, wherein the DNA template is selected from a T1 to T8 template as detailed in FIG 4A to 4D, or wherein the DNA template comprises a sequence as defined by any of SEQ ID NOs: 1 to 19, and/or the complement thereof, or a sequence having at least 99% identity to the respective sequence.

3. The molecular complex according to claims 1 and 2, wherein Base¹ and Base² of the Cap analog as defined in claim 1 (i) are independently selected from adenine or an analog of adenine, Base³ is guanine or an analog of guanine, and
wherein the DNA template as defined in claim 1 (ii) comprises a promoter with a core region with a sequence as defined by any one of SEQ ID NO: 1 to 3, or wherein the initiating sequence is selected from an AGG, an AAG, an ATG, a GGG, a GG, an AGA or a GAG.

4. The molecular complex according to any one of the preceding claims, wherein Base¹ is independently selected from any one of adenine, N6-benzyladenine, N6-methyladenine, N6-(2-phenylethyl)adenine, and R1 and/or R2 are independently O-Me, and
(i) Base² and Base³ are bothguanine, or
(ii) Base² is adenine and Base³ is guanine, or
(iii) Base² and Base³ are independently guanine or adenine, or
(iv) Base² is adenine and Base³ is uracil, or
(v) Base² is uracil and Base³ is guanine.
in the Cap analog as defined in claim 1.

5. The molecular complex according to any one the preceding claims, additionally comprising a T7 RNA polymerase and/or a homolog thereof and/or a modified variant thereof.

6. A vector, or more than one vector, wherein the at least one vector comprises the at least one DNA template as defined in claims 1, 2 or 3.

7. A DNA molecule, or more than one than one DNA molecule, wherein the at least one DNA molecule comprises the at least one DNA template as defined in claims 1, 2, or 3.

8. A use of the molecular complex according to any one of the preceding claims for in vitro transcription, wherein the capping efficiency is at least 80%, 81%, 82%, 83%, 84%, 85%, 86%, 87%, 88%, 89%, 90%, 91%, 92%, 93%, 94%, 95%, 96%, 97%, 98%, 99%, or up to 100%.

9. A method of producing at least one m7G-capped mRNA molecule, the method comprising:
(i) providing a reaction mixture comprising the Cap analog as defined in claims 1, 3 and 4, the DNA template as defined in claims 1 to 3 and/or the vector according to claim 6 and/or the DNA molecule according to claim 7, and at least one T7 RNA polymerase and/or the sequence encoding the same;
(ii) allowing in vitro transcription from said DNA template and/or from said vector and obtaining at least one m7G-capped mRNA;
(iv) optionally: purifying said at least one m7G-capped mRNA; and/or
(v) optionally: formulating said at least one m7G-capped mRNA molecule yielding a pharmaceutical composition.

10. The method of claim 9 further comprising a step of:
(vii) introducing or contacting said at least one m7G-capped mRNA molecule obtained in step (iii) into or with at least one target cell in an in vitro cellular system for transfecting said at least one target cell and/or for in vitro studying the functionality of said at least one m7G-capped mRNA.

11. A pharmaceutical composition for use in a method of preventing and/or treating a disease, the pharmaceutical composition comprising the at least one m7G-capped mRNA molecule obtained or obtainable by the method according to claim 9 or 10, additionally comprising at least one pharmaceutically acceptable carrier or excipient.

12. The pharmaceutical composition for use in a method of preventing and/or treating a disease according to claim 11, the pharmaceutical composition additionally comprising at least one lipid nanoparticle, wherein the at least one lipid nanoparticle is selected from the group of at least one liposome (LPs), at least one liposome-like nanoparticle (LLP), at least one solid lipid nanoparticle (SLN), at least one nanostructured lipid carrier (NLC), at least one lipid-polymer hybrid nanoparticle (LPN), at least one lipoprotein particle (LPT), at least one nanoemulsion, at least one cationic nanoemulsion (CNE) and at least one exosome.

13. A kit comprising the Cap analog as defined in claims 1, 3 or 4, optionally comprising the DNA template as defined in claims 1 to 3, and/or the vector according to claim 6 and/or the DNA molecule according to claim 7, optionally wherein the kit comprises at least one further reagent and/or at least one aqueous solution, preferably a buffer.
